# FASCICULE DE BREVET EUROPEEN

(11) **EP 0 792 883 B1**
(45) Date de publication et mention de la délivrance du brevet: **20.12.2000**
(21) Numéro de dépôt: 97400435.0
(22) Date de dépôt: 27.02.1997
(51) Int. Cl.: C07F 5/02, A61K 31/69

(54) **Nouveaux dérivés de l'acide boronique, leur procédé de préparation et les compositions pharmaceutiques qui les contiennent**
Neue Boronsäure-Derivate, Verfahren zu ihrer Herstellung und sie enthaltende Arzneimittel
Novel derivates of boronic acid, process for their preparation and pharmaceutical compositions containing them

(30) Priorité: 27.02.1996 FR 9602377
(43) Date de publication de la demande: 03.09.1997
(73) Titulaire: ADIR ET COMPAGNIE, 92415 Courbevoie Cédex (FR)
(72) Inventeur: de Nanteuil, Guillaume, 92150 Suresnes (FR); Gloanec, Philippe, 78380 Bougival (FR); Lila, Christine, 78220 Viroflay (FR); Portevin, Bernard, 78990 Elancourt (FR); Verbeuren, Tony, 78540 Vernouillet (FR); Rupin, Alain, 37510 Savonnieres (FR); Simonet, Serge, 78700 Conflans Sainte Honorine (FR)

(56) Documents cités:
- EP-A- 0 293 881
- WO-A-94/21650

## Description

La présente invention concerne des nouveaux dérivés d'acide boronique, leur procédé de préparation, les compositions pharmaceutiques qui les contiennent ainsi que leur utilisation en tant qu'inhibiteurs de trypsine-like serine proteases.

L'une de ces serine proteases, la thrombine, est l'enzyme clé de la coagulation et joue un rôle central dans la pathologie des thromboses veineuses et artérielles comme l'ont montré F. Toti et coll. (Sang, Thrombose, Vaisseaux, 4, 483-494, 1992) et T.M. REILLY et coll. (Blood Coagulation and Fibrinolysis, 3, 513-517, 1992).

Les approches anti-thrombotiques sont plus efficaces et sans risque par rapport aux traitements actuels. Des inhibiteurs directs de la thrombine, actuellement en développement clinique, présentent toute une série d'avantages sur l'héparine. Mais ces substances, l'hirudine et l'hirulog-1 ont le désavantage de ne pas être actives par voie orale.

D'autre part, il est connu que des peptides contenant la séquence (D)Phe-Pro-Arg sont des inhibiteurs du site catalytique de la thrombine (C. KETTNER et coll., J. Biol. Chem., 265 (30), 18289-18297, 1990).

Des dérivés peptidiques de l'acide boronique, présentant une activité anti-thrombotique, ont déjà été décrits dans la littérature. C'est le cas plus particulièrement des composés décrits dans les brevets WO 94 21650, EP 239881 et EP 471651. M.A. HUSSAIN et coll. ont d'ailleurs démontré que l'acide Ac-(D)Phe-Pro-Arg boronique (DUP 714) est un inhibiteur de la thrombine (Peptides, 12, 1153-1154, 1991).

Il était donc particulièrement intéressant de synthétiser de nouveaux inhibiteurs de serine proteases afin d'augmenter la puissance et la sélectivité des composés déjà décrits dans la littérature. De plus, ces composés, qui ne sont plus des dérivés peptidiques, présentent différents temps de coagulation augmentés ainsi qu'une activité par voie orale.

Plus spécifiquement, la présente invention concerne les composés de formule (I): 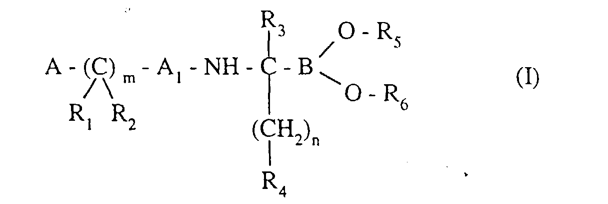 dans laquelle :
- R₁, R₂,: identiques ou différents, représentent un atome d'hydrogène, ou un groupement alkyle (C₁-C₆) linéaire ou ramifié ou bien R₁ et R₂ forment avec l'atome de carbone qui les porte un groupement cycloalkyle (C₃-C₈),
- R₃: représente un atome d'hydrogène ou un groupement alkyle (C₁-C₆) linéaire ou ramifié, un groupement phényle éventuellement substitué ou un groupement benzyle éventuellement substitué,
- R₄: représente :
- un groupement amino substitué éventuellement par un ou plusieurs groupements, identiques ou différents, alkyle (C₁-C₆) linéaire ou ramifié, benzyle éventuellement substitué, aryle ou hétérocyclique,
- un groupement amidino substitué éventuellement par un ou plusieurs groupements, identiques ou différents, alkyle (C₁-C₆) linéaire ou ramifié, benzyle éventuellement substitué, aryle ou hétérocyclique,
- un groupement guanidino substitué éventuellement par un groupement alkyle (C₁-C₆) linéaire ou ramifié, benzyle éventuellement substitué, aryle ou hétérocyclique, - isothiouréïdo substitué éventuellement par un groupement alkyle (C₁-C₆) linéaire ou ramifié, benzyle éventuellement substitué, aryle ou hétérocyclique,
- iminométhylamino éventuellement substitué par un groupement alkyle (C₁-C₆) linéaire ou ramifié,
- mercapto substitué par un groupement hétérocyclique,
- ou, un groupement hétérocyclique,
- R₅, R₆: réprésentent chacun un atome d'hydrogène ou un groupement alkyle (C₁-C₆) linéaire ou ramifié, ou 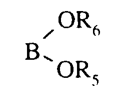 forme un ester boronique de pinanediol,
- m: représente un entier tel que 0 ≤ m ≤ 6,
- n: représente un entier tel que 1 ≤ n ≤ 6,
- A: représente l'un quelconque des groupements suivants :
* un groupement bicycloalkyle (C₅-C₁₀) phényle substitué éventuellement par un ou plusieurs, identiques ou différents, atomes d'halogène ou groupements alkyle (C₁-C₆) linéaire ou ramifié, alkoxy (C₁-C₆) linéaire ou ramifié, hydroxy ou amino (éventuellement substitué par un ou deux groupements, identiques ou différents, alkyle (C₁-C₆) linéaire ou ramifié, alkylsulfonyle (C₁-C₆) linéaire ou ramifié ou arylsulfonyle),
* ou, un groupement de formule : 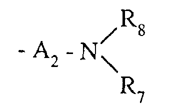 à la condition que, dans ce cas, m soit différent de zéro,
   dans laquelle :

R₇, R₈, identiques ou différents, représentent un atome d'hydrogène, un groupement alkyle (C₁-C₆) linéaire ou ramifié (éventuellement substitué par un ou plusieurs groupements aryle, hétérocyclique, arylsulfonylamino, bicycloalkyle (C₅-C₁₀) phényle éventuellement substitué par un ou plusieurs groupements tels que définis plus haut ou indanyle), un groupement alkylsulfonyle (C₁-C₆) linéaire ou ramifié, un groupement arylsulfonyle, un groupement aryle, un groupement hétérocyclique ou un groupement bicycloalkyle (C₅-C₁₀) phényle (éventuellement substitué par un ou plusieurs groupements tels que définis plus haut), un groupement indanyle ou l'un quelconque des groupements : 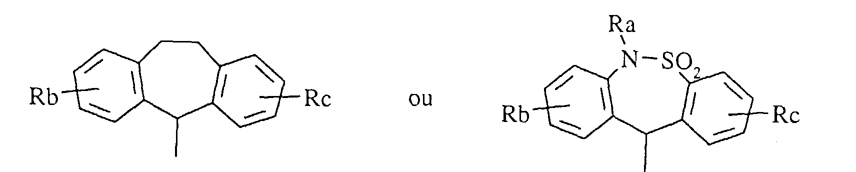 dans lesquels :
Ra représente un groupement alkyle (C₁-C₆) linéaire ou ramifié ou phényle éventuellement substitué,
Rb, Rc, identiques ou différents, représentent un atome d'hydrogène, d'halogène, ou un groupement alkyle (C₁-C₆) linéaire ou ramifié, alkoxy (C₁-C₆) linéaire ou ramifié, hydroxy, amino substitué ou non, ou trihalogénométhyle,

A₂ représente -CO- ou -CS-,
- A₁: représente -CO-, -CS- ou -SO₂-,
leurs isomères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, benzène sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Par groupement aryle, on entend phényle, naphtyle, tétrahydronaphtyle, chacun de ces groupements étant éventuellement substitué par un ou plusieurs atomes d'halogène ou groupement alkyle (C₁-C₆) linéaire ou ramifié, cycloalkyle (C₃-C₇), bicycloalkyle (C₅-C₁₀), alkoxy (C₁-C₆) linéaire ou ramifié, hydroxy ou trihalogénométhyle, ou amino (substitué éventuellement par un ou plusieurs groupements alkyle (C₁-C₆) linéaire ou ramifié).

Par groupement hétérocycle, on entend un groupement mono ou bicyclique, saturé ou insaturé, de 5 à 12 chaînons contenant un, deux ou trois hétéroatomes choisis parmi oxygène, azote ou soufre, étant entendu que l'hétérocycle peut être éventuellement subsitué par un ou plusieurs atomes d'halogène ou groupement alkyle (C₁-C₆) linéaire ou ramifié, alkoxy (C₁-C₆) linéaire ou ramifié, hydroxy, trihalogénométhyle, amino (substitué éventuellement par un ou plusieurs groupements alkyle (C₁ -C₆) linéaire ou ramifié), imino ou arylsulfonyle.

Par groupement phényle ou benzyle éventuellement substitué, on entend éventuellement substitué par un ou plusieurs, identiques ou différents, atomes d'halogène ou groupements alkyle (C₁-C₆) linéaire ou ramifié, alkoxy C₁-C₆) linéaire ou ramifié, hydroxy, amino substitué ou non, trihalogénoalkyle (C₁-C₆) linéaire ou ramifié.

L'invention concerne préférentiellement les composés de formule (I) dans laquelle A₁ représente -CO-, R₃ représente un atome d'hydrogène et R₄ représente un groupement guanidino éventuellement substitué.

Dans la définition de A, un groupement bicycloalkyle (C₅-C₁₀) phényle éventuellement substitué est préférentiellement le groupement (bicyclo[2.2.2]oct-1-yl)phényle éventuellement substitué.
Lorsque A représente un groupement bicycloalkyle (C₅-C₁₀) phényle éventuellement substitué, m est préférentiellement égal à 0.
Lorsque A représente le groupement 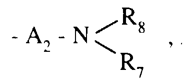 A₂ est préférentiellement -CO-, R₇ est préférentiellement hydrogène et R₈ est préférentiellement un groupement alkyle (C₁-C₆) linéaire ou ramifié, substitué préférentiellement par un ou plusieurs groupements aryle.
Lorsque A représente un groupement 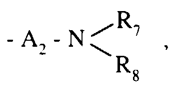 m est préférentiellement égal à 1 et R₁, R₂ forment avec l'atome de carbone qui les porte un groupement cycloalkyle (C₃-C₇). Un groupement cycloalkyle préféré pour la définition de 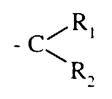 est le groupement cyclopentyle.

L'invention s'étend également au procédé de préparation des composés de formule (I) caractérisé en ce que l'on met en réaction un composé de formule (II) : 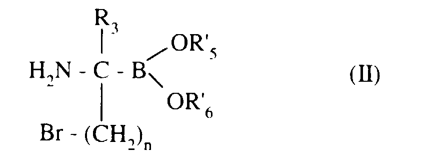 dans laquelle R₃ et n ont la même signification que dans la formule (I), R'₅ et R'₆ représentent chacun un groupement alkyle (C₁-C₆) linéaire ou ramifié ou 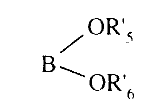 forme un ester boronique de pinanediol,
avec un composé de formule (III): 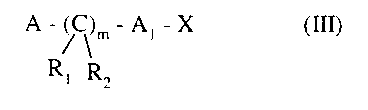 dans laquelle A, A₁, R₁, R₂ et m ont la même signification que dans la formule (I),
X représente un atome de chlore ou un groupement hydroxy,
pour conduire au composé de formule (IV) : 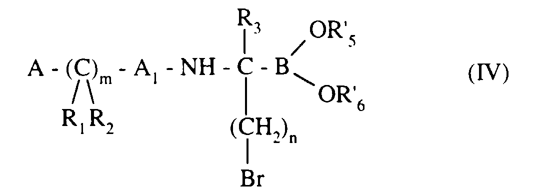 dans laquelle A, A₁, R₁, R₂, R₃, R'₅, R'₆, m et n ont la même signification que précédemment, composé de formule (IV) qui peut être transformé selon la nature du groupement R₄ que l'on souhaite obtenir :
- **soit**, en dérivé cyano correspondant par action du cyanure de cuivre puis, réaction en milieu alcoolique en présence d'acide suivi de l'action de l'ammoniaque, en dérivé amidino correspondant de formule (I/a), cas particulier des composés de formule (I): 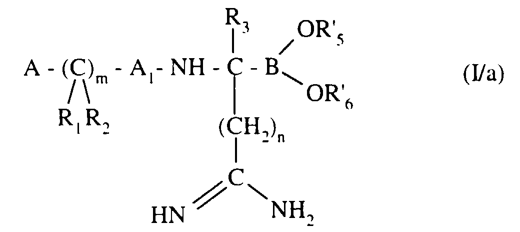 dans laquelle A, A₁, R₁, R₂, R₃, R'₅, R'₆, m et n ont la même signification que précédemment, dont on substitue, si on le désire, la fonction amidino,
- **soit**, en dérivé azido correspondant par action de l'azoture de sodium, puis par hydrogénation catalytique en dérivé amino correspondant de formule (I/b), cas particulier des composés de formule (I): 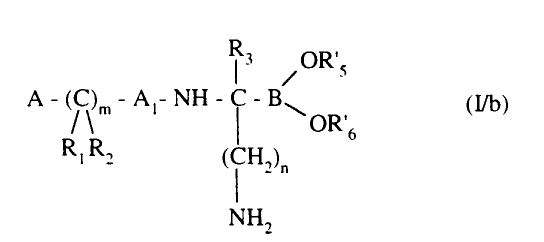 dans laquelle A, A₁, R₁, R₂, R₃, R'₅, R'₆, m et n ont la même signification que précédemment, composé de formule (I/b) dont on substitue, si on le désire, la fonction amine,
   et dont on transforme, si on le désire, le groupement amino :
   - en *groupement guanidino* par réaction avec du cyanamide,
      pour conduire au composé de formule (I/c), cas particulier des composés de formule (I) : 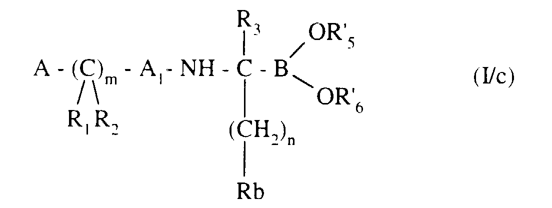 dans laquelle A, A₁, R₁, R₂, R₃, R'₅, R'₆, m et n ont la même signification que précédemment et Rb représente un groupement guanidino, dont on substitue, si on le désire, la fonction guanidino,
   - ou, en *groupement iminométhylamino* par réaction avec du formimidate d'éthyle, pour conduire au composé de formule (I/d): 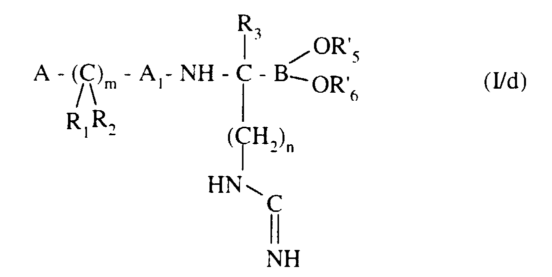 dans laquelle A, A₁, R₁, R₂, R₃, R'₅, R'₆, m et n ont la même signification que précédemment, dont on substitue, si on le désire, la fonction aminido,
- **soit**, par réaction avec une thiourée éventuellement substituée,
   pour conduire au composé de formule (I/e), cas particulier des composés de formule (I) : 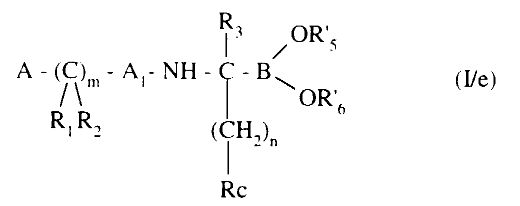 dans laquelle A, A₁, R₁, R₂, R₃, R'₅, R'₆, m et n ont la même signification que précédemment, et Rc représente un groupement isothiouréïdo éventuellement substitué,
- **soit**, par réaction avec un hétérocycle, convenablement protégé, une amine substituée par un hétérocycle convenablement protégé ou un thiol substitué par un hétérocycle convenablement protégé,
   pour conduire, après déprotection, au composé de formule (I/f), cas particulier des composés de formule (I) : 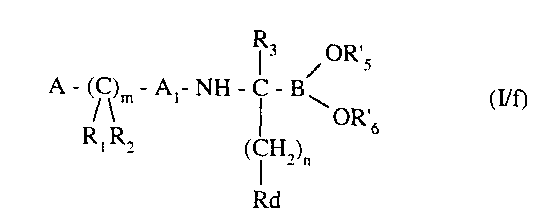 dans laquelle A, A₁, R₁, R₂, R₃, R'₅, R'₆, m et n ont la même signification que précédemment, et Rd représente un hétérocycle, un groupement amino substitué par un hétérocycle ou un groupement mercapto substitué par un hétérocycle,
composés de formule (I/a), (I/b), (I/c), (I/d), (I/e) ou (I/f), que l'on transforme, si on le souhaite, à l'aide de trichlorure de bore ou d'acide phénylboronique, en acide boronique correspondant de formule (I/g) : 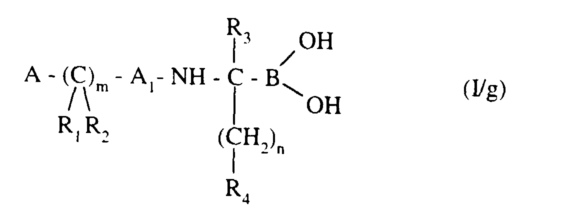 dans laquelle A, A₁, R₁, R₂, R₃, R₄, m et n ont la même signification que dans la formule (I),
composés de formule (I/a) à (I/g) :
- qui peuvent être, le cas échéant, purifiés selon une technique classique de purification,
- dont on sépare, le cas échéant, les isomères selon une technique classique de séparation,
- que l'on transforme, si on le souhaite, en ses sels d'addition à un acide ou à une base pharmaceutiquement acceptable.

Les composés de formule (II) sont obtenus :
- *soit* à partir du composé de formule (V) obtenu selon le procédé décrit par M.W. RATHKE et coll. (J. Organomet. Chem., 122, 145-149, 1976) : 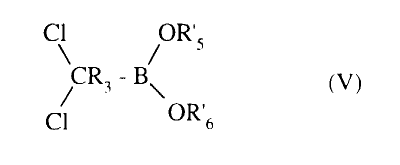 dans laquelle R₃, R'₅ et R'₆ sont tels que définis plus haut,
   que l'on fait réagir sur un organomagnésium de formule (VI) :

   Br(CH₂)ₙ - MgCl (VI)

   dans laquelle n, R₃, R'₅, R'₆ sont tels que définis précédemment, que l'on met en réaction avec le 1,1,1,3,3,3-hexaméthyldisilazane (HMDS) en présence de n-butyllithium, pour conduire, après traitement en milieu acide, au composé de formule (II),
- *soit* à partir d'un ester boronique α-chloré de formule (VII), préparé selon le procédé décrit par D.S. MATTESON et coll. (Organometallics, 3, 1284-1288, 1984) et W. RATHKE et coll. (J. Biol. Chem., 265 (30), 18289-18297, 1990) : 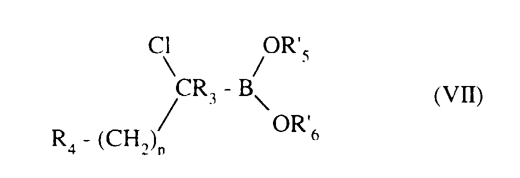 dans laquelle R₃, R'₅, R'₆ et n sont tels que définis plus haut, que l'on met en réaction avec le 1,1,1,3,3,3-hexaméthyldisilazane (HMDS) en présence de n-butyllithium pour conduire, après traitement en milieu acide, au composé de formule (II).

Le composé de formule (II) peut également être obtenu selon le procédé décrit par D.S. MATTESON et coll. (Organometallics, 3, 1284-1288, 1984) et W. RATHKE et coll. (J. Biol. Chem., 265 (30), 18289-18297, 1990).

Les composés de formule (I/b) substitués ou non sur la fonction amine peuvent également être obtenus par réaction d'une benzylamine éventuellement substituée sur le composé de formule (IV).

Les composés de la présente invention, outre le fait qu'ils soient nouveaux, présentent des propriétés pharmacologiques particulièrement intéressantes.

Ce sont de puissants inhibiteurs de trypsine-like serine protéases qui présentent une importante sélectivité vis-à-vis de la thrombine par rapport à d'autres serine protéases de la coagulation et de la fibrinolyse. Ils possèdent d'autre part une meilleure activité par voie orale que le composé de référence DUP 714.

Ces propriétés les rendent donc utiles dans le traitement des angines stables ou non, des maladies d'origine thrombotique et/ou donnant lieu à des complications thrombotiques ainsi que dans le traitement ou la prévention de l'infarctus du myocarde et des thromboses veineuses ou artérielles ainsi que pour la prévention de la coagulation du sang lorsqu'il est en contact avec des cathéters ou des réservoirs.

Ils peuvent également être utilisés en association thérapeutique avec un thrombolytique.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc...

La posologie utile est adaptable selon la nature et la sévérité de l'affection, la voie d'administration ainsi que selon l'âge et le poids du patient. Cette posologie varie de 1 à 500 mg par jour en une ou plusieurs prises.

Les exemples suivants illustrent l'invention mais ne la limitent en aucune façon.

Les produits de départ utilisés sont des produits de départ connus ou préparés selon des modes opératoires connus.

Les préparations A à Z et AA à AK conduisent à des intermédiaires de synthèse, utiles pour la préparation des composés de l'invention.

Les structures des composés décrits dans les exemples ont été déterminées selon les techniques spectrophotométriques usuelles (infrarouge, RMN, spectrométrie de masse, ...)

### Préparation A: Chlorure de l'acide 4-(4-méthoxybicyclo[2.2.2]oct-1-yl)benzoïque

*Stade A : Acide 4-(4-méthoxybicyclo[2.2.2]oct-1-yl)benzoïque*
Le produit attendu est obtenu selon le procédé décrit dans le brevet EP 599 732.
*Point de fusion* : *239°C*
*Stade B* : *Chlorure de l'acide 4-(4-méthoxybicyclo[2.2.2]oct-1-yl)benzoique*
A 10 mmoles de l'acide obtenu au stade précédent en solution dans du tétrachlorure de carbone à 0°C, sont ajoutés 10 mmoles de pentachlorure de phosphore. Après retour à température ambiante et 18 heures d'agitation, la solution est évaporée. Le résidu est repris par du dichlorométhane. Le produit attendu est obtenu après évaporation et séchage.

| *Microanalyse élémentaire* : | | | |
|---|---|---|---|
| | *C %* | *H %* | *N %* |
| *calculé* | *68,94* | *6,87* | *12,72* |
| *trouvé* | *68,38* | *6,94* | *12,91* |

### Préparation B : Chlorure de l'acide 4-(4-chlorobicyclo[2.2.2]oct-1-yl)benzoïque

Le produit attendu est obtenu selon le procédé décrit dans la préparation A.
*Stade A : Acide 4-(4-chlorobicyclo[2.2.2]oct-1-yl)benzoïque*
   *Point de fusion : > 260°C*
*Stade B : Chlorure de l'acide 4-(4-chlorobicyclo[2.2.2]oct-1-yl)benzoïque*
   *Point de fusion* : *128°C*

### Préparation C : Chlorure de l'acide [4-(4-méthoxybicyclo[2.2.2]oct-1-yl]phénylacétique

*Stade A : Acide [4-(4-méthoxybicyclo[2.2.2]oct-1-yl]phénylacétique*
Le produit attendu est obtenu selon le procédé décrit dans le brevet EP 599 732.
*Point de fusion : 220°C*

| *Microanalyse élémentaire :* | | |
|---|---|---|
| | *C %* | *H %* |
| *calculé* | *74, 42* | *8,08* |
| *trouvé* | *74,49* | *8,13* |

*Stade B: Chlorure de l'acide [4-(4-méthoxybicyclo[2.2.2]oct-1-yl]phénylacétique*
Le chlorure d'acide correspondant est obtenu selon le procédé décrit au stade B de la préparation A.

### Préparation D : Chlorure de l'acide 4-(4-hydroxybicyclo[2.2.2]oct-1-yl)benzoïque

Le produit attendu est obtenu selon le procédé décrit dans la préparation A.
*Stade A : Acide 4-(4-hydroxybicyclo[2.2.2]oct-1-ylbenzoïque*
*Stade B : Chlorure de l'acide 4-(4-hydroxybicyclo[2.2.2]oct-1-yl)benzoïque*

### Préparation E : Chlorure de l'acide 4-(2,4-diméthoxybicyclo[2.2.2]oct-1-yl)benzoïque

Le produit attendu est obtenu selon le procédé décrit dans la préparation A.
*Stade A : Acide 4-(2,4-diméthoxybicyclo[2.2.2]oct-1-yl)benzoïque*
*Stade B : Chlorure de l'acide 4-(2,4-diméthoxybicyclo[2.2.2]oct-1-yl)benzoïque*

| *Microanalyse élémentaire :* | | | |
|---|---|---|---|
| | *C %* | *H%* | *Cl %* |
| *calculé* | *66,12* | *6,85* | *11,48* |
| *trouvé* | *65,58* | *6,75* | *12,23* |

### Préparation F : Acide 1-benzylaminocarbonylcyclopentanecarboxylique

### Stade A: Monoester éthylique de l'acide cyclopentane-1,1-dicarboxylique

A 480 mmoles de diisopropylamine dans 600 ml de tétrahydrofurane anhydre (THF), sous argon, à -70°C sont ajoutées 480 mmoles de butyllithium. après 15 minutes d'agitation à -70°C, 240 mmoles d'acide cyclopentane carboxylique sont ajoutées goutte à goutte. Après retour à température ambiante, l'ensemble est chauffé une heure à 50°C. Le milieu réactionnel est alors refroidi à -70°C et 260 mmoles de chloroformiate d'éthyle sont additionnées. La solution est agitée 20 minutes puis versée dans de l'eau glacée. Le milieu est acidifié par de l'acide chlorhydrique concentré puis extrait au dichlorométhane. La phase organique est alors lavée à l'eau jusqu'à pH neutre puis par une solution saturée de chlorure de sodium. Après séchage et évaporation, on obtient le produit attendu sous forme d'huile.
*Infrarouge (nujol) :* ν_{CO} = *1702 cm*^{*-1*}

### Stade B : Monoester éthylique de l'acide 1-benzylaminocarbonylcyclopentanecarboxylique

A une solution contenant 50 mmoles du composé obtenu au stade précédent, 59 mmoles de benzylamine et 59 mmoles de diisopropyléthylamine dans 100 ml de dichlorométhane anhydre, sont ajoutées 59 mmoles de tétrafluoroborate d'0-benzothiazol-1-yl-N,N,N',N'-tétraméthyluronium. Le milieu est maintenu sous agitation une nuit à température ambiante. Après évaporation du solvant, reprise par de l'acétate d'éthyle, la phase organique est lavée, séchée et évaporée et conduit au produit attendu sous forme d'huile.

### Stade C : Acide 1-benzylaminocarbonylcyclopentanecarboxylique

A une solution contenant 55 mmoles du composé obtenu au stade précédent dans 150 ml de THF sont ajoutées 110 ml de soude 1N. Après 5 heures à température ambiante, le THF est évaporé. La phase aqueuse est acidifiée puis extraite à l'acétate d'éthyle. La phase organique est alors séchée puis évaporée. Le produit attendu est alors obtenu par recristallisation du résidu dans de l'éther isopropylique.
*Point de fusion : 92-94°C*

Les préparations G à K ont été réalisées selon le procédé décrit dans la préparation F à partir des produits de départ correspondants :

### Préparation G : Acide 1-benzylaminocarboxylcyclobutanecarboxylique

*Point de fusion : 106-108°C*

### Préparation H : Acide 1-benzylaminocarbonylcyclohexanecarboxylique

*Point de fusion* *: 126-128°C*

### Préparation I : Acide 1-benzylaminocarbonylcyclopropanecarboxylique

*Infrarouge (nujol) :* ν_{CO} = *1710 cm*^{*-1*}

### Préparation J : Acide 2-benzylaminocarbonyl-2-méthylpropionique

*Point de fusion* *: 112-114°C*

### Préparation K : Acide 2-benzylaminocarbonyl-2-éthylbutyrique

*Point de fusion : 132-134°C*

### Préparation L : Chlorure de l'acide 3-(4-méthoxybicyclo[2.2.2]oct-1-yl)benzoïque

### Préparation M : Chlorure de l'acide 4-(4-éthoxybicyclo[2.2.2]oct-1-yl)benzoïque

Le produit attendu est obtenu selon le procédé décrit dans la préparation A.
*Stade A : Acide 4-(4-éthoxybicyclo[2.2.2]oct-1-yl)benzoïque*
*Stade B : Chlorure de l'acide 4-(4-éthoxybicyclo[2.2.2]oct-1-yl)benzoïque*

### Préparation N : Chlorure de l'acide 4-(4-hydroxybicyclo[2.2.2]oct-1-yl)benzoïque

Le produit attendu est obtenu selon le procédé décrit dans la préparation A.
*Stade A : Acide 4-(4-hydroxybicyclo[2.2.2]oct-1-yl)benzoïque*
*Stade B : Chlorure de l'acide 4-(4-hydroxybicyclo[2.2.2]oct-1-yl)benzoïque*

### Préparation O : Chlorure de l'acide 4-(4-isopropylbicyclo[2.2.2]oct-1-yl)benzoïque

Le produit attendu est obtenu selon le procédé décrit dans la préparation A.
*Stade A : Acide 4-(4-isopropylbicyclo[2.2.2]oct-1-yl)benzoïque*
*Stade B : Chlorure de l'acide 4-(4-isopropylbicyclo[2.2.2]oct-1-yl)benzoïque*

### Préparation P : Chlorure de l'acide 4-(bicyclo[2.2.2]oct-1-yl)benzoïque

Le produit attendu est obtenu selon le procédé décrit dans la préparation A.

Les préparations Q à Z et AA à AK ont été réalisées selon le procédé décrit dans la préparation F à partir des produits de départ correspondants.

### Préparation Q : Acide 1-phénéthylaminocarbonylcyclopentanecarboxylique

### Préparation R : Acide 1-(N-méthylphénéthylaminocarbonyl)cyclopentanecarboxylique

### Préparation S : Acide 1-(3,5-bistrifluorométhylbenzylaminocarbonyl)cyclopentane carboxylique

### Préparation T : Acide 1-(3-méthoxyphénéthylaminocarbonyl)cyclopentanecarboxylique

### Préparation U : Acide 1-(4-hydroxyphénéthylaminocarbonyl)cyclopentanecarboxylique

### Préparation V : Acide 1-(3,4-diméthoxybenzylaminocarbonyl)cyclopentanecarboxylique

### Préparation W : Acide 1-(3,4-diméthoxyphénéthylaminocarbonyl)cyclopentane carboxylique

### Préparation X : Acide 1-[2-(pyridin-3-yl)-éthylaminocarbonyl]cyclopentane carboxylique

### Préparation Y : Acide 1-(benzhydrylaminocarbonyl)cyclopentanecarboxylique

### Préparation Z : Acide 1-(2,2-diphényléthylaminocarbonyl)cyclopentanecarboxylique

### Préparation AA : Acide 1-(naphtalèn-2-yl-méthylaminocarbonyl)cyclopentane carboxylique

### Préparation AB : Acide 1-[2-(naphtalèn-2-yl)-éthylaminocarbonyl]cyclopentane carboxylique

### Préparation AC : Acide 1-(1,1-diméthyl-2-phényléthylaminocarbonyl)cyclopentane carboxylique

### Préparation AD : Acide 1-(3-phénylpropylaminocarbonyl)cyclopentanecarboxylique

### Préparation AE : Acide 1-(2-benzènesulfonylaminoéthylaminocarbonyl)cyclopentane carboxylique

### Préparation AF : Acide 1-[4-(4-méthoxybicyclo[2,2.2]oct-1-yl)benzylaminocarbonyl] cyclopentane carboxylique

### Préparation AG : Acide 1-(indan-2-yl-aminocarbonyl)cyclopentanecarboxylique

### Préparation AH : Acide 1-(indan-2-yl-méthylaminocarbonyl)cyclopentanecarboxylique

### Préparation AI : Acide 1-(10,11 -dihydro-5H-dibenzo[a,d]cyclohepten-5-yl-aminocarbonyl)cyclopentane carboxylique

### Préparation AJ : Acide 1-(6,11-dihydro-6-méthyl-dibenzo[c,f][1,2]thiazèpin-11-yl-aminocarbonyl)cyclopentanecarboxylique

### Préparation AK : Acide 1-(1-benzènesulfonyl-pipéridin-4-yl-aminocarbonyl) cyclopentane carboxylique

### Préparation AL : Chlorure de l'acide 2-(4-méthoxybicyclo[2.2.2]-phényl]-2-méthyl propanoïque

Le produit attendu est obtenu selon le procédé décrit dans la préparation A.

### Exemple 1 : 1-(R)-[4-(4-Méthoxybicyclo[2.2.2]oct-1-yl)benzoylamino]-4-aminobutyl boronate de (+)α-pinanediol, chlorhydrate

### Stade A : 1-(R)-[4-(4-Méthoxybicyclo[2.2.2]oct-1-yl)benzoylamino]-4-bromobutyl boronate de (+)α-pinanediol

A 20 mmoles du chlorure d'acide décrit dans la préparation A dans 130 ml de dichlorométhane anhydre, sont ajoutées 20 mmoles de 1-(R)-amino-4-bromobutyl boronate de (+)α-pinanediol décrit dans le brevet EP 615 978. Le milieu réactionnel est refroidi à -20°C et 44 mmoles de triéthylamine sont ajoutées goutte à goutte. Après retour à température ambiante et agitation pendant 18 heures, l'ensemble est évaporé. Le résidu est repris avec un mélange eau/acétate d'éthyle. La phase organique est récupérée puis séchée avec une solution aqueuse saturée d'hydrogénocarbonate de sodium, de l'eau, de l'acide citrique à 10 % puis d'une solution saturée de chlorure de sodium. Après séchage et évaporation, on obtient le produit attendu.

### Stade B : 1-(R)-[4-(4-Méthoxybicyclo[2.2.2]oct-1-yl)benzoylamino]-4-azidobutyl boronate de (+ )α-pinanediol

15 mmoles du produit obtenu au stade précédent dans 30 ml de diméthylformamide anhydre sont placées à 100°C en présence de 30 mmoles d'azoture de sodium pendant 4 heures. Après 12 heures à température ambiante, l'ensemble est repris par un mélange acétate d'éthyle/eau, la phase organique est lavée plusieurs fois à l'eau, séchée et évaporée et conduit au produit attendu.

### Stade C : 1 -(R)-[4-(4-Méthoxybicyclo[2.2.2]oct-1-yl)benzoylamino]-4-aminobutyl boronate de (+)α-pinanediol, chlorhydrate

14 mmoles du composé obtenu au stade précédent dans 250 ml de méthanol anhydre sont hydrogénées en présence de 2 mmoles de chloroforme en utilisant 50 mg de palladium/C à 10% comme catalyseur, pendant 2 heures. Après filtration du catalyseur, rinçage et évaporation, on obtient le produit attendu.

### Exemple 2 : 1-(R)-[4-(4-Méthoxybicyclo[2.2.2]oct-1-yl)benzoylamino]-4-guanidinobutyl boronate de (+)α-pinanediol, chlorhydrate

10 mmoles du composé obtenu dans l'exemple 1 et 100 mmoles de cyanamide sont portées à reflux dans 80 ml d'éthanol anhydre pendant 2 jours. Après évaporation de l'éthanol, passage sur résine sephadex en reprenant le résidu par du méthanol, on obtient le produit attendu.

### Exemple 3 : Acide 1-(R)-[4-(4-méthoxybicyclo[2.2.2]oct-1-yl)benzoylamino]-4-guanidinobutyl boronique, chlorhydrate

A 10 mmoles du composé obtenu dans l'exemple 2 en suspension dans 150 ml d'éther éthylique, sont ajoutées 48 mmoles d'acide phénylboronique et 150 ml d'acide chlorhydrique 1N. Le milieu est fortement agité à température ambiante puis la phase aqueuse est décantée, lavée à l'éther puis amenée à sec. Le produit attendu est obtenu après purification du résidu par passage sur résine Bio-gel P₂ en utilisant comme éluant un mélange acide chlorhydrique 0,001N/acétonitrile (1/1).
*Spectre de masse :* FAB⁺ : *[M+H]*⁺ : *m/z* = *417*

### Exemple 4 : 1-(R)-[4-(4-Chlorobicyclo[2.2.2]oct-1-yl)benzoylamino]-4-aminobutyl boronate de (+)α-pinanediol, chlorhydrate

Le produit attendu est obtenu selon le procédé décrit dans l'exemple 1 en utilisant comme produit de départ le composé décrit dans la préparation B.

### Exemple 5 : 1-(R)-[4-(4-Chlorobicyclo[2.2.2]oct-1-yl)benzoylamino]-4-guanidinobutyl boronate de (+)α-pinanediol, chlorhydrate

Le produit attendu est obtenu selon le procédé décrit dans l'exemple 2 à partir du composé décrit dans l'exemple 4.

### Exemple 6 : Acide 1-(R)-[4-(4-chlorobicyclo[2.2.2]oct-1-yl)benzoylamino ]-4-guanidinobutyl boronique, chlorhydrate

Le produit attendu est obtenu selon le procédé décrit dans l'exemple 3 à partir du composé décrit dans l'exemple 5.
*Spectre de masse : FAB*⁺ *: [M+H]*⁺ *: m/z = 421*

### Exemple 7 : 1-(R)-{[4-(4-Méthoxybicyclo[2.2.2]oct-1-yl)phényl]acétylamino}-4-aminobutyl boronate de (+)α-pinanediol, chlorhydrate

Le produit attendu est obtenu selon le procédé décrit dans l'exemple 1 en utilisant comme produit de départ le composé décrit dans la préparation C.

### Exemple 8 : 1-(R)-{[4-(4-Méthoxybicyclo[2.2.2]oct-1-yl)phényl]acétylamino}-4-guanidinobutyl boronate de (+)α-pinanediol, chlorhydrate

Le produit attendu est obtenu selon le procédé décrit dans l'exemple 2 à partir du composé décrit dans l'exemple 7.

### Exemple 9 : Acide 1-(R)-{[4-(4-méthoxybicyclo[2.2.2]oct-1-yl)phényl]acétylamino}-4-guanidinobutyl boronique, chlorhydrate

Le produit attendu est obtenu selon le procédé décrit dans l'exemple 3 à partir du composé décrit dans l'exemple 8.
*Spectre de masse : FAB*⁺ *: [M+H]*⁺ : *m/z = 431*

### Exemple 10 : 1-(R)-[4-(4-Hydroxybicyclo[2.2.2]oct-1-yl)benzoylamino]-4-aminobutyl boronate de (+)α-pinanediol, chlorhydrate

Le produit attendu est obtenu selon le procédé décrit dans l'exemple 1 en utilisant comme produit de départ le composé décrit dans la préparation D.

### Exemple 11 : 1-(R)-[4-(4-Hydroxybicyclo[2.2,2]oct-1-yl)benzoylamino]-4-guanidino butyl boronate de (+)α-pinanediol, chlorhydrate

Le produit attendu est obtenu selon le procédé décrit dans l'exemple 2 à partir du composé décrit dans l'exemple 10.

### Exemple 12 : Acide 1-(R)-[4-(4-hydroxybicyclo[2.2.2]oct-1-yl)benzoylamino]-4-guanidino butyl boronique, chlorhydrate

Le produit attendu est obtenu selon le procédé décrit dans l'exemple 3 à partir du composé décrit dans l'exemple 11.
*Spectre de masse : FAB*⁺ *: [M+H]*⁺ *: m/z = 403*

### Exemple 13 : 1-(R)-[2,4-Diméthoxybicyclo[2.2.2]oct-1-yl)benzoylamino]-4-aminobutyl boronate de (+)α-pinanediol, chlorhydrate

Le produit attendu est obtenu selon le procédé décrit dans l'exemple 1 en utilisant comme produit de départ le composé décrit dans la préparation E.

### Exemple 14 : 1-(R)-[2,4-Diméthoxybicyclo[2.2.2]oct-1-yl)benzoylamino]-4-guanidinobutyl boronate de (+)α-pinanediol, chlorhydrate

Le produit attendu est obtenu selon le procédé décrit dans l'exemple 2 en utilisant comme produit de départ le composé décrit dans l'exemple 13.

### Exemple 15 : Acide 1-(R)-[4-(2,4-diméthoxybicyclo[2.2.2]oct-1-yl)benzylamino]-4-guanidinobutyl boronique, chlorhydrate

Le produit attendu est obtenu selon le procédé décrit dans l'exemple 3 en utilisant comme produit de départ le composé décrit dans l'exemple 14.
*Spectre de masse : FAB*⁺ : *[M+H]*⁺*:m/z = 447*

### Exemple 16 : 1-(R)-[4-(4-Méthoxybicyclo[2.2.2]oct-1-yl)benzoylamino]-4-(N-méthylamino)butyl boronate de (+)α-pinanediol, benzène sulfonate

A 1 mmole du composé décrit dans l'exemple 1 dans 20 ml d'éthanol anhydre sont ajoutés 5,7 g de tamis moléculaire 3Å et 3,75 ml d'une solution aqueuse de formaldéhyde à 40 %. L'ensemble est agité une nuit à température ambiante. Après filtration, 1 mmole d'acide benzène sulfonique est ajoutée aux phases éthanoliques et l'ensemble est hydrogéné en présence de 100 mg de Pd/C à 10 % comme catalyseur pendant une nuit à température ambiante et pression atmosphérique. Le produit attendu est obtenu après filtration du catalyseur et purificiation sur résine Sephadex^{®}.

### Exemple 17 : Acide 1-(R)-[4-(4-méthoxybicyclo[2.2.2]oct-1-yl)benzoylamino]-4-(N-méthylamino)butyl boronique

Le produit attendu est obtenu selon le procédé décrit dans l'exemple 3 à partir du composé décrit dans l'exemple 16.

### Exemple 18 : 1-(R)-[4-(4-Chlorobicyclo[2.2.2]oct-1-yl)benzoylamino]-4-(isothiouréïdo-2)butyl boronate de (+)α-pinanediol

### Stade A : 1-(R)-[4-(4-Chlorobicyclo[2.2.2]oct-1-yl)benzoylamino]-4-bromobutyl boronate de (+)α-pinanediol

Le produit attendu est obtenu selon le procédé décrit au stade A de l'exemple 1 en utilisant comme produit de départ le composé décrit dans la préparation B.

### Stade B : 1-(R)-[4-(4-Chlorobicyclo[2.2.2]oct-1-yl)benzoylamino]-4-(isothiouréïdo-2)butyl boronate de (+)α-pinanediol

2,4 mmoles du composé obtenu au stade précédent et 7,3 mmoles de thiourée dans 6 ml d'éthanol sont agitées 60 heures à température ambiante. Après évaporation du solvant, le produit attendu est obtenu après purification par passage sur résine "Sephadex^{®}" en utilisant le méthanol comme éluant.

### Exemple 19 : Acide 1-(R)-[4-(4-chlorobicyclo[2.2.2]oct-1-yl)benzoylamino-4-(isothiouréïdo-2)butyl boronique, chlorhydrate

Le produit attendu est obtenu selon le procédé décrit dans l'exemple 3 à partir du composé décrit dans l'exemple 18.
*Spectre de masse : FAB*⁺ *: [M+H]*⁺ *: m/z = 438*

### Exemple 20 : 1-(R)-(1-Benzylaminocarbonylcyclopentanecarboxamido)-4-aminobutyl boronate de (+)α-pinanediol, benzène sulfonate

### Stade A : 1-(R)-(1-Benzylaminocarbonylcylopentanecarboxamido)-4-bromobutyl boronate de (+)α-pinanediol

A 10 mmoles du composé décrit dans la préparation F dans 50 ml de THF anhydre sont ajoutées 10 mmoles de N-méthylmorpholine. L'ensemble est placé sous argon à -20°C, puis 10 mmoles de chloroformiate d'isobutyle sont ajoutées et l'ensemble est agité 20 minutes. 10 mmoles de 1-(R)-amino-4-bromobutyl boronate de (+)α-pinanediol (décrit dans le brevet EP 615 978) dans 50 ml de THF anhydre sont alors ajoutées à -20°C puis goutte à goutte 20 mmoles de triéthylamine. Après une heure à -20°C, puis 12 heures à température ambiante, le THF est évaporé. Le résidu est repris par de l'acétate d'éthyle. La phase organique est lavée, séchée et évaporée. Le produit attendu est obtenu sous forme d'huile après purification sur colonne de silice en utilisant comme éluant un mélange dichlorométhane/acétate d'éthyle (8/2).

### Stade B : 1-(R)-(1-Benzylaminocarbonylcylopentanecarboxamido)-4-azidobutyl boronate de (+)α-pinanediol

Le produit attendu est obtenu selon le procédé décrit au stade B de l'exemple 1 à partir du composé décrit au stade précédent.

### Stade C : 1-(R)-(1-Benzylaminocarbonylcylopentanecarboxamido)-4-aminobutyl boronate de (+)α-pinanediol, benzène sulfonate

Le produit attendu est obtenu selon le procédé décrit au stade C de l'exemple 1 à partir du composé décrit au stade précédent et en utilisant de l'acide benzène sulfonique à la place du chloroforme.

### Exemple 21 : 1-(R)-(1-Benzylaminocarbonylcyclopentanecarboxamido)-4-guanidinobutyl boronate de (+)α-pinanediol, benzène sulfonate

Le produit attendu est obtenu selon le procédé décrit dans l'exemple 2 à partir du composé décrit dans l'exemple 20.

### Exemple 22 : Acide 1-(R)-(1-benzylaminocarbonylcyclopentanecarboxamido)-4-guanidinobutyl boronique, benzène sulfonate

7 mmoles du composé obtenu dans l'exemple 21 dans un mélange eau/éther (1/1) en présence de 7 mmoles d'acide benzène sulfonique et 30 mmoles d'acide phényl boronique sont agitées pendant une nuit. La phase aqueuse est décantée, concentrée et le produit attendu est obtenu après purification sur résine BIOGEL en utilisant comme éluant un mélange acétonitrile/eau (1/1).
*Spectre de masse : FAB*⁺ *: [M+H]*⁺ : *m/z = 404*

### Exemple 23 : 1-(R)-(1-Benzylaminocarbonylcyclobutanecarboxamido)-4-aminobutyl boronate de (+)α-pinanediol, benzène sulfonate

Le produit attendu est obtenu selon le procédé décrit dans l'exemple 20 en utilisant comme produit de départ le composé décrit dans la préparation G.

### Exemple 24 : 1-(R)-(1-Benzylaminocarbonylcyclobutanecarboxamido)-4-guanidinobutyl boronate de (+)α-pinanediol, benzène sulfonate

Le produit attendu est obtenu selon le procédé décrit dans l'exemple 2 à partir du composé décrit dans l'exemple 23.

### Exemple 25 : Acide 1-(R)-(1-benzylaminocarbonylcyclobutanecarboxamido)-4-guanidinobutyl boronique, benzène sulfonate

Le produit attendu est obtenu selon le procédé décrit dans l'exemple 22 à partir du composé décrit dans l'exemple 24.
*Spectre de masse : FAB*⁺ *: [M+H]*⁺ : *m/z = 390*

### Exemple 26 : 1-(R)-(1-Benzylaminocarbonylcyclohexanecarboxamido)-4-aminobutyl boronate de (+)α-pinanediol, benzène sulfonate

Le produit attendu est obtenu selon le procédé décrit dans l'exemple 20 en utilisant comme produit de départ le composé décrit dans la préparation H.

### Exemple 27 : 1-(R)-(1-Benzylaminocarbonylcyclohexanecarboxamido)-4-guanidinobutyl boronate de (+)α-pinanediol, benzène sulfonate

Le produit attendu est obtenu selon le procédé décrit dans l'exemple 2 à partir du composé décrit dans l'exemple 26.

### Exemple 28 : Acide 1-(R)-(1-benzylaminocarbonylcyclohexanecarboxamido)-4-guanidinobutyl boronique, benzène sulfonate

Le produit attendu est obtenu selon le procédé décrit dans l'exemple 22 à partir du composé décrit dans l'exemple 27.
*Spectre de masse : FAB*⁺ *: [M+H]*⁺ : *m/z = 418*

### Exemple 29 : 1-(R)-(1-Benzylaminocarbonylcyclopropanecarboxamido)-4-aminobutylboronate de (+)α-pinanediol, benzène sulfonate

Le produit attendu est obtenu selon le procédé décrit dans l'exemple 20 en utilisant comme produit de départ le composé décrit dans la préparation I.

### Exemple 30 : 1-(R)-(1-Benzylaminocarbonylcyclopropanecarboxamido)-4-guanidinobutyl boronate de (+)α-pinanediol, benzène sulfonate

Le produit attendu est obtenu selon le procédé décrit dans l'exemple 2 à partir du composé décrit dans l'exemple 29.

### Exemple 31 : Acide 1-(R)-(1-benzylaminocarbonylcyclopropanecarboxamido)-4-guanidinobutyl boronique, benzène sulfonate

Le produit attendu est obtenu selon le procédé décrit dans l'exemple 3 à partir du composé décrit dans l'exemple 30.
*Spectre de masse : FAB*⁺ : *[M+H]*⁺ : *m/z = 376*

### Exemple 32 : 1-(R)-(2-Benzylaminocarbonyl-2-méthylpropionamido)-4-aminobutyl boronate de (+)α-pinanediol, benzène sulfonate

Le produit attendu est obtenu selon le procédé décrit dans l'exemple 20 en utilisant comme produit de départ le composé décrit dans la préparation J.

### Exemple 33 : 1-(R)-(2-Benzylaminocarbonyl-2-méthylpropionamido)-4-guanidinobutyl boronate de (+)α-pinanediol, benzène sulfonate

Le produit attendu est obtenu selon le procédé décrit dans l'exemple 2 à partir du composé décrit dans l'exemple 32.

### Exemple 34 : Acide 1-(R)-(2-benzylaminocarbonyl-2-méthylpropionamido)-4-guanidinobutyl boronique, benzène sulfonate

Le produit attendu est obtenu selon le procédé décrit dans l'exemple 22 à partir du composé décrit dans l'exemple 33.
*Spectre de masse : FAB*⁺ *: [M+glycerol-2H*_{*2*}*O+H*⁺*]: m/z = 434*

### Exemple 35 : 1-(R)-(2-Benzylaminocarbonyl-2-éthylbutylamido)-4-aminobutyl boronate de (+)α-pinanediol, benzène sulfonate

Le produit attendu est obtenu selon le procédé décrit dans l'exemple 20 en utilisant comme produit de départ le composé décrit dans la préparation K.

### Exemple 36 : 1-(R)-(2-Benzylaminocarbonyl-2-éthylbutylamido)-4-guanidinobutyl boronate de (+)α-pinanediol, benzène sulfonate

Le produit attendu est obtenu selon le procédé décrit dans l'exemple 2 à partir du composé décrit dans l'exemple 35.

### Exemple 37 : Acide 1-(R)-(2-benzylaminocarbonyl-2-éthylbutylamido)-4-guanidinobutyl boronique, benzène sulfonate

Le produit attendu est obtenu selon le procédé décrit dans l'exemple 22 à partir du composé décrit dans l'exemple 36.
*Spectre de masse :* FAB⁺ : *[M+H]*⁺ : *m/z = 406*

### Exemple 38 : Acide 1-(R)-[4-(4-méthoxybicyclo[2.2.2]oct-1-yl)benzoylamino]-4-aminobutyl boronique, chlorhydrate

Le produit attendu est obtenu selon le procédé décrit dans l'exemple 3, à partir du composé décrit dans l'exemple 1.

### Exemple 39 : Acide 1-(R)-[4-(4-chlorobicyclo[2.2.2]oct-1-yl)benzoylamino]-4-aminobutyl boronique, chlorhydrate

Le produit attendu est obtenu selon le procédé décrit dans l'exemple 3 à partir du composé décrit dans l'exemple 4.

### Exemple 40 : Acide 1-(R)-{[4-(4-méthoxybicyclo[2.2.2]oct-1-yl)phényl]-acétylamino}-4-aminobutyl boronique, chlorhydrate

Le produit attendu est obtenu selon le procédé décrit dans l'exemple 3 à partir du composé décrit dans l'exemple 7.

### Exemple 41 : Acide 1-(R)-[4-(4-hydroxybicyclo[2.2.2]oct-1-yl)benzoylamino]-4-aminobutyl boronique, chlorhydrate

Le produit attendu est obtenu selon le procédé décrit dans l'exemple 3 à partir du composé décrit dans l'exemple 10.

### Exemple 42 : Acide 1-(R)-[4-(2,4-diméthoxybicyclo[2.2.2]oct-1-yl)benzylamino]-4-aminobutyl boronique, chlorhydrate

Le produit attendu est obtenu selon le procédé décrit dans l'exemple 3 à partir du composé décrit dans l'exemple 13.

### Exemple 43 : 1-(R)-[4-(4-Méthoxybicyclo[2.2.2]oct-1-yl)benzoylamino]-4-(isothiouréïdo-2)butyl boronate de (+)α-pinanediol

Le produit attendu est obtenu selon le procédé décrit dans l'exemple 18 à partir du composé décrit dans la préparation A.

### Exemple 44 : Acide 1-(R)-[4-(4-méthoxybicyclo[2.2.2]oct-1-yl)benzoylamino]-4-(isothiouréïdo-2)butyl boronique, chlorhydrate

Le produit attendu est obtenu selon le procédé décrit dans l'exemple 3 à partir du composé décrit dans l'exemple 43.

### Exemple 45 : 1-(R)-[4-(4-Méthoxybicyclo[2.2.2]oct-1-yl)benzylamino]-5-aminopentyl boronate de (+)α-pinanediol, chlorhydrate

Le produit attendu est obtenu selon le procédé décrit dans l'exemple 1 en utilisant comme produits de départ le composé décrit dans la préparation A et le 1-(R)-amino-5-bromopentylboronate de (+)α-pinanediol décrit dans le brevet EP 615 978.

### Exemple 46 : Acide 1-(R)-[4-(4-méthoxybicyclo[2.2.2]oct-1-yl)benzoylamino]-5-aminopentyl boronique, chlorhydrate

Le produit attendu est obtenu selon le procédé décrit dans l'exemple 3 à partir du composé décrit dans l'exemple 45.

### Exemple 47 : 1-(R)-[4-(4-Méthoxybicyclo[2.2.2]oct-1-yl)benzoylamino]-5-guanidinopentylboronate de (+)α-pinanediol, chlorhydrate

Le produit attendu est obtenu selon le procédé décrit dans l'exemple 2 à partir du composé décrit dans l'exemple 45.

### Exemple 48 : Acide 1-(R)-[4-(4-méthoxybicyclo[2.2.2]oct-1-yl)benzoylamino]-5-guanidinopentylboronique, chlorhydrate

Le produit attendu est obtenu selon le procédé décrit dans l'exemple 3 à partir du composé décrit dans l'exemple 47.

### Exemple 49 : 1(R)-[3-(4-Méthoxybicyclo[2.2.2]oct-1-yl)benzoylamino]-4-aminobutyl boronate de (+)α-pinanediol, chlorhydrate

Le produit attendu est obtenu selon le procédé décrit dans l'exemple 1 en utilisant comme produit de départ le composé décrit dans la préparation L.

### Exemple 50 : :1-(R)-[3-(4-Méthoxybicyclo[2.2.2]oct-1-yl)benzoylamino]-4-guanidinobutyl boronate de (+)α-pinanediol, chlorhydrate

Le produit attendu est obtenu selon le procédé décrit dans l'exemple 2 à partir du composé décrit dans l'exemple 49.

### Exemple 51 : Acide 1-(R)-[3-(4-méthoxybicyclo[2.2.2]oct-1-yl)benzoylamino]-4-guanidinobutylboronique, chlorhydrate

Le produit attendu est obtenu selon le procédé décrit dans l'exemple 3 à partir du composé décrit dans l'exemple 50.

### Exemple 52 : 1-(R)-[4-(4-Ethoxybicyclo[2.2.2]oct-1-yl)benzoylamino]-4-aminobutyl boronate de (+)α-pinanediol, chlorhydrate

Le produit attendu est obtenu selon le procédé décrit dans l'exemple 1 en utilisant comme produit de départ le composé décrit dans la préparation M.

### Exemple 53 : 1-(R)-[4-(4-Ethoxybicyclo[2.2.2]oct-1-yl)benzoylamino]-4-guanidinobutyl boronate de (+)α-pinanediol, chlorhydrate

Le produit attendu est obtenu selon le procédé décrit dans l'exemple 2 à partir du composé décrit dans l'exemple 52.

### Exemple 54 : Acide 1-(R)-[4-(4-éthoxybicyclo[2.2.2]oct-1-yl)benzoylamino]-4-guanidinobutyl boronique, chlorhydrate

Le produit attendu est obtenu selon le procédé décrit dans l'exemple 3 à partir du composé décrit dans l'exemple 53.

### Exemple 55 : 1-(R)-[4-(4-Méthoxybicyclo[2.2.2]oct-1-yl)benzoylamino]-4-(N-méthylguanidino)butyl boronate de (+)α-pinanediol, chlorhydrate

Le produit attendu est obtenu selon le procédé décrit dans l'exemple 2 à partir du composé décrit dans l'exemple 16.

### Exemple 56 : Acide 1-(R)-[4-(4-méthoxybicyclo[2,2.2]oct-1-yl)benzoylamino]-4-(N-méthylguanidino)butyl boronique, chlorhydrate

Le produit attendu est obtenu selon le procédé décrit dans l'exemple 3 à partir du composé décrit dans l'exemple 55.

### Exemple 57 : 1-(R)-[4-(4-Méthoxybicyclo[2.2.2]oct-1-yl)benzoylamino]-4-(iminométhylamino)butylboronate de (+)α-pinanediol, chlorhydrate

Le produit attendu est obtenu en faisant réagir le formimidate d'éthyle, selon le procédé décrit dans le brevet PCT/US 94/04058, avec le composé décrit dans l'exemple 1.

### Exemple 58 : Acide 1-(R)-[4(-4-méthoxybicyclo[2.2.2]oct-1-yl)benzoylamino]-4-(iminométhylamino)butyl boronique, chlorhydrate

Le produit attendu est obtenu selon le procédé décrit dans l'exemple 3 à partir du composé décrit dans l'exemple 57.

### Exemple 59 : 1-(R)-[4-(4-Méthoxybicyclo[2.2.2]oct-1-yl)benzoylamino]-4-[iminométhyl(N-méthyl)amino]butyl boronate de (+)α-pinanediol, chlorhydrate

Le produit attendu est obtenu selon le procédé décrit dans l'exemple 57 à partir du composé décrit dans l'exemple 16.

### Exemple 60 : Acide 1-(R)-[4-(4-méthoxybicyclo[2.2.2]oct-1-yl)benzoylamino]-4-[iminométhyl-(N-méthyl)amino]butyl boronique, chlorhydrate

Le produit attendu est obtenu selon le procédé décrit dans l'exemple 3 à partir du composé décrit dans l'exemple 59.

### Exemple 61 : 1-(R)-[4-(4-Méthoxybicyclo[2.2.2]oct-1-yl)benzoylamino]-4-(isothiouréïdo-2)butylboronate de (+)α-pinanediol

Le produit attendu est obtenu selon le procédé décrit dans l'exemple 18, stade B, à partir du composé décrit dans l'exemple 1, stade A.

### Exemple 62 : Acide 1-(R)-[4-(4-méthoxybicyclo[2.2.2]oct-1-yl)benzoylamino]-4-(isothiouréïdo-2)butyl boronique, chlorhydrate

Le produit attendu est obtenu selon le procédé décrit dans l'exemple 3 à partir du composé décrit dans l'exemple 61.

### Exemple 63 : 1-(R)-[4-(4-Chlorobicyclo[2.2.2]oct-1-yl)benzoylamino]-5-aminopentyl boronate de (+)a-pinanediol, chlorhydrate

Le produit attendu est obtenu selon le procédé décrit dans l'exemple 45, en utilisant comme produit de départ le composé décrit dans la préparation B.

### Exemple 64 : Acide 1-(R)-[4-(4-chlorobicyclo[2.2.2]oct-1-yl)benzoylamino]-5-aminopentyl boronique, chlorhydrate

Le produit attendu est obtenu selon le procédé décrit dans l'exemple 3 à partir du composé décrit dans l'exemple 63.

### Exemple 65 : 1-(R)-[4-(4-Chlorobicyclo[2.2.2]oct-1-yl)benzoylamino]-5-guanidinopentyl boronate de (+)a-pinanediol, chlorhydrate

Le produit attendu est obtenu selon le procédé décrit dans l'exemple 2 à partir du composé décrit dans l'exemple 63.

### Exemple 66 : Acide 1-(R)-[4-(4-chlorobicyclo[2.2.2]oct-1-yl)benzoylamino]-5-guanidinopentyl boronique, chlorhydrate

Le produit attendu est obtenu selon le procédé décrit dans l'exemple 3, à partir du composé décrit dans l'exemple 65.

### Exemple 67 : 1-(R)-[3-(4-Hydroxybicyclo[2.2.2]oct-1-yl)benzoylamino]-4-aminobutyl boronate de (+)α-pinanediol,chlorhydrate

Le produit attendu est obtenu selon le procédé décrit dans l'exemple 1 en utilisant comme produit de départ le composé décrit dans la préparation N.

### Exemple 68 : 1-(R)-[3-(4-Hydroxybicyclo[2.2.2]oct-1-yl)benzoylamino]-4-guanidinobutyl boronate de (+)α-pinanediol, chlorhydrate

Le produit attendu est obtenu selon le procédé décrit dans l'exemple 2, à partir du composé décrit dans l'exemple 67.

### Exemple 69 : Acide 1-(R)-[3-(4-hydroxybicyclo[2.2.2]oct-1-yl)benzoylamino]-4-guanidinobutyl boronique, chlorhydrate

Le produit attendu est obtenu selon le procédé décrit dans l'exemple 3 à partir du composé décrit dans l'exemple 68.

### Exemple 70 : 1-(R)-[4-(4-Isopropylbicyclo[2.2.2]oct-1-yl)benzoylamino]-4-aminobutyl boronate de (+ )α-pinanediol, chlorhydrate

Le produit attendu est obtenu selon le procédé décrit dans l'exemple 1 en utilisant comme produit de départ le composé décrit dans la préparation O.

### Exemple 71 : 1-(R)-[4-(4-Isopropylbicyclo[2.2.2]oct-1-yl)benzoylamino]-4-guanidinobutyl boronate de (+)α-pinanediol, chlorhydrate

Le produit attendu est obtenu selon le procédé décrit dans l'exemple 2 à partir du composé décrit dans l'exemple 70.

### Exemple 72 : Acide 1-(R)-[4-(4-Isopropylbicyclo[2.2.2]oct-1-yl)benzoylamino]-4-guanidinobutyl boronique, chlorhydrate

Le produit attendu est obtenu selon le procédé décrit dans l'exemple 3 à partir du composé décrit dans l'exemple 71.

### Exemple 73 : 1-(R)-[4-(bicyclo[2.2.2]oct-1-yl)benzoylamino]-4-aminobutyl boronate de (+)α-pinanediol, chlorhydrate

Le produit attendu est obtenu selon le procédé décrit dans l'exemple 1 en utilisant comme produit de départ le composé décrit dans la préparation P.

### Exemple 74 : 1-(R)-[4-(bicyclo[ 2.2.2]oct-1-yl)benzoylamino]-4-guanidinobutyl boronate de (+)α-pinanediol, chlorhydrate

Le produit attendu est obtenu selon le procédé décrit dans l'exemple 2 à partir du composé décrit dans l'exemple 73.

### Exemple 75 : Acide 1-(R)-[4-(bicyclo[2.2.2]oct-1-yl)benzoylamino]-4-guanidinobutyl boronique, chlorhydrate

Le produit attendu est obtenu selon le procédé décrit dans l'exemple 3 à partir du composé décrit dans l'exemple 74.

### Exemple 76 : 1-(R)-[4-(4-Méthoxybicyclo[2.2.2]oct-1-yl)benzoylamino]-4-1-méthylimidazol-2-yl)thiobutyl boronate de (+)α-pinanediol

Le produit attendu est obtenu en faisant réagir le 1-méthyl-2-mercaptoimidazole, selon le procédé décrit dans le brevet EP 401 462, avec le composé décrit dans l'exemple 1, stade A.

### Exemple 77 : Acide 1-(R)-[4-(4-méthoxybicyclo[2.2.2]oct-1-yl)benzoylamino]-4-(1-méthylimidazol-2-yl)thiobutyl boronique, chlorhydrate

Le produit attendu est obtenu selon le procédé décrit dans l'exemple 3 à partir du composé décrit dans l'exemple 76.

### Exemple 78 : 1-(R)-(1-Phénéthylaminocarbonylcyclopentanecarboxamido)-5-aminopentyl boronate de (+)α-pinanediol, chlorhydrate

Le produit attendu est obtenu selon le procédé décrit dans l'exemple 20 en utilisant comme produits de départ le composé décrit dans la préparation Q et le 1-(R)-amino-5-bromopentyl boronate de (+)α-pinanediol décrit dans le brevet EP 615 978, et en remplaçant l'acide benzène sulfonique par du chloroforme.

### Exemple 79 : Acide 1-(R)-(1-phénéthylaminocarbonylcyclopentanecarboxamido)-5-aminopentyl boronique, chlorhydrate

Le produit attendu est obtenu selon le procédé décrit dans l'exemple 3 à partir du composé décrit dans l'exemple 78.

### Exemple 80 : 1-(R)-(1-Phénéthylaminocarbonylcyclopentanecarboxamido)-4-aminobutyl boronate de (+)α-pinanediol, chlorhydrate

Le produit attendu est obtenu selon le procédé décrit dans l'exemple 20 en utilisant comme produit de départ le composé décrit dans la préparation Q et en remplaçant au stade C l'acide benzène sulfonique par le chloroforme.

### Exemple 81 : Acide 1-(R)-(1-Phénéthylaminocarbonylcyclopentanecarboxamido)-4-aminobutyl boronique, chlorhydrate

Le produit attendu est obtenu selon le procédé décrit dans l'exemple 3 à partir du composé décrit dans l'exemple 80.

### Exemple 82 : 1-(R)-(1-Phénéthylaminocarbonylcyclopentanecarboxamido)-4-guanidinobutyl boronate de (+)α-pinanediol, benzène sulfonate

Le produit attendu est obtenu selon le procédé décrit dans l'exemple 2 à partir du composé décrit dans l'exemple 80 sous forme de benzène sulfonate.

### Exemple 83 : Acide 1-(R)-(1-phénéthylaminocarbonylcyclopentanecarboxamido)-4-guanidinobutyl boronique, benzène sulfonate

Le produit attendu est obtenu selon le procédé décrit dans l'exemple 22 à partir du composé décrit dans l'exemple 82.

### Exemple 84 : 1-(R)-(1-Phénéthylaminocarbonylcyclopentanecarboxamido)-4-(4-imino-4H-pyridin-1-yl)butyl boronate de (+)α-pinanediol, chlorhydrate

### Stade A : 1-(R)-(1-Phénéthylaminocarbonylcyclopentanecarboxamido)-4-bromobutyl boronate de (+)α-pinanediol

Le produit attendu est obtenu selon le procédé décrit dans l'exemple 20, stade A, en utilisant comme produit de départ le composé décrit dans la préparation Q.

### Stade B : 1-(R)-(1-Phénéthylaminocarbonylcyclopentanecarboxamido)-4-(4-imino-4H-pyridin-1-yl)-butyl boronate de (+)α-pinanediol, chlorhydrate

Le produit attendu est obtenu selon le procédé décrit au stade B de l'exemple 1 à partir du composé décrit au stade précédent, en utilisant la 4-aminopyridine à la place de l'azoture de sodium.

### Exemple 85 : Acide 1-(R)-(1-phénétylaminocarbonylcyclopentanecarboxamido)-4-(4-imino-4H-pyridin-1-yl)-butyl boronique, chlorhydrate

Le produit attendu est obtenu selon le procédé décrit dans l'exemple 3 à partir du composé décrit dans l'exemple 84.

### Exemple 86 : 1-(R)-[1-(N-Méthyl-phénéthylaminocarbonyl)cyclopentanecarboxamido]-4-aminobutyl boronate de (+)α-pinanediol, benzène sulfonate

Le produit attendu est obtenu selon le procédé décrit dans l'exemple 20 en utilisant comme produit de départ le composé décrit dans la préparation R.

### Exemple 87 : 1-(R)-[1-(N-Méthyl-phénéthylaminocarbonyl)cyclopentanecarboxamido]-4-guanidinobutyl boronate de (+)α-pinanediol, benzène sulfonate

Le produit attendu est obtenu selon le procédé décrit dans l'exemple 2, à partir du composé décrit dans l'exemple 86.

### Exemple 88 : Acide 1-(R)-[1-(N-méthyl-phénéthylaminocarbonyl)cyclopentane carboxamido]-4-guanidinobutyl boronique, benzène sulfonate

Le produit attendu est obtenu selon le procédé décrit dans l'exemple 22 à partir du composé décrit dans l'exemple 87.

### Exemple 89 : 1(R)-[1-(3,5-bistrifluorométhylbenzylaminocarbonyl) cyclopentanecarboxamido]-4-aminobutyl boronate (+)α-pinanediol, benzène sulfonate

Le produit attendu est obtenu selon le procédé décrit dans l'exemple 20 en utilisant comme produit de départ le composé décrit dans la préparation S.

### Exemple 90 : 1-(R)-[1-(3,5-bistrifluorométhylbenzylaminocarbonyl)cyclopentanecarboxamido]-4-guanidinobutyl boronate de (+)α-pinanediol, benzène sulfonate

Le produit attendu est obtenu selon le procédé décrit dans l'exemple 2 à partir du composé décrit dans l'exemple 89.

### Exemple 91 : Acide 1-(R)-[1-(3,5-bistrifluorométhylbenzylaminocarbonyl)-cyclopentane carboxamido]-4-guanidinobutyl boronique, benzène sulfonate

Le produit attendu est obtenu selon le procédé décrit dans l'exemple 22 à partir du composé décrit dans l'exemple 90.

### Exemple 92 : 1-(R)-[1-(3-Méthoxyphénéthylaminocarbonyl)cyclopentanecarboxamido]-4-aminobutyl boronate de (+)α-pinanediol, benzène sulfonate

Le produit attendu est obtenu selon le procédé décrit dans l'exemple 20 en utilisant comme produit de départ le composé décrit dans la préparation T.

### Exemple 93 : 1-(R)-[1-(3-Méthoxyphénéthylaminocarbonyl)cyclopentanecarboxamido]-4-guanidinobutyl boronate de (+)α-pinanediol, benzène sulfonate

Le produit attendu est obtenu selon le procédé décrit dans l'exemple 2 à partir du composé décrit dans l'exemple 92.

### Exemple 94 : Acide 1-(R)-[1-(3-méthoxyphénéthylaminocarbonyl)cyclopentane carboxamido]-4-guanidinobutyl boronique, benzène sulfonate

Le produit attendu est obtenu selon le procédé décrit dans l'exemple 22 à partir du composé décrit dans l'exemple 93.

### Exemple 95 : 1-(R)-[1-(4-Hydroxyphénéthylaminocarbonyl)cyclopentanecarboxamido]-4-aminobutyl boronate de (+)α-pinanediol, benzène sulfonate

Le produit attendu est obtenu selon le procédé décrit dans l'exemple 20 en utilisant comme produit de départ le composé décrit dans la préparation U.

### Exemple 96 : 1-(R)-[1-(4-Hydroxyphénéthylaminocarbonyl)cyclopentanecarboxamido]-4-guanidinobutyl boronate de (+)α-pinanediol benzène sulfonate

Le produit attendu est obtenu selon le procédé décrit dans l'exemple 2 à partir du composé décrit dans l'exemple 95.

### Exemple 97 : Acide 1-(R)-[1-(4-hydroxyphénéthylaminocarbonyl)cyclopentane carboxamido]-4-guanidinobutyl boronique, benzène sulfonate

Le produit attendu est obtenu selon le procédé décrit dans l'exemple 22 à partir du composé décrit dans l'exemple 96.

### Exemple 98 : 1-(R)-[1-(3,4-Diméthoxybenzylaminocarbonyl)cyclopentanecarboxamido]-4-aminobutyl boronate de (+)α-pinanediol, benzène sulfonate

Le produit attendu est obtenu selon le procédé décrit dans l'exemple 20 en utilisant comme produit de départ le composé décrit dans la préparation V.

### Exemple 99 : 1-(R)-[1-(3,4-Diméthoxybenzylaminocarbonyl)cyclopentanecarboxamido]-4-guanidinobutyl boronate de (+)α-pinanediol, benzène sulfonate

Le produit attendu est obtenu selon le procédé décrit dans l'exemple 2 à partir du composé décrit dans l'exemple 98.

### Exemple 100 : Acide 1-(R)-[1-(3,4-diméthoxybenzylaminocarbonylcyclopentane carboxamido]-4-guanidinobutyl boronique, benzène sulfonate

Le produit attendu est obtenu selon le procédé décrit dans l'exemple 22 à partir du composé décrit dans l'exemple 99.

### Exemple 101 : 1-(R)-[1-(3,4-Diméthoxyphénéthylaminocarbonyl)cyclopentane carboxamido]-4-aminobutyl boronate de (+)α-pinanediol, benzène sulfonate

Le produit attendu est obtenu selon le procédé décrit dans l'exemple 20 en utilisant comme produit de départ le composé décrit dans la préparation W.

### Exemple 102 : 1-(R)-[1-(3,4-Diméthoxyphénéthylaminocarbonyl)cyclopentane carboxamido]-4-guanidinobutyl boronate de (+)α-pinanediol, benzène sulfonate

Le produit attendu est obtenu selon le procédé décrit dans l'exemple 2 à partir du composé décrit dans l'exemple 101.

### Exemple 103 : Acide 1-(R)-[1-(3,4-diméthoxyphénéthylaminocarbonyl)cyclopentane carboxamido]-4-guanidinobutyl boronique, benzène sulfonate

Le produit attendu est obtenu selon le procédé décrit dans l'exemple 22 à partir du composé décrit dans l'exemple 102.

### Exemple 104 : 1-(R)-{1-[2-(Pyridin-3-yl)-éthylaminocarbonyl]cyclopentanecarboxamido}-4-aminobutyl boronate de (+)α-pinanediol, dichlorhydrate

Le produit attendu est obtenu selon le procédé décrit dans l'exemple 20, en utilisant comme produit de départ le composé décrit dans la préparation X et en remplaçant l'acide benzène sulfonique par du chloroforme.

### Exemple 105 : 1-(R)-{1-[2-(Pyridin-3-yl)-éthylaminocarbonyl]cyclopentanecarboxamido}-4-guanidinobutyl boronate de (+)α-pinanediol. dichlorhydrate

Le produit attendu est obtenu selon le procédé décrit dans l'exemple 2 à partir du composé décrit dans l'exemple 104.

### Exemple 106 : Acide 1-(R)-{1-[2-(pyridin-3-yl)-éthylaminocarbonyl]cyclopentane carboxamido}-4-guanidinobutyl boronique, dichlorhydrate

Le produit attendu est obtenu selon le procédé décrit dans l'exemple 3 à partir du composé décrit dans l'exemple 105.

### Exemple 107 : 1-(R)-1-(Benzhydrylaminocarbonylcyclopentanecarboxamido)-4-aminobutyl boronate de (+)α-pinanediol, benzène sulfonate

Le produit attendu est obtenu selon le procédé décrit dans l'exemple 20 en utilisant comme produit de départ le composé décrit dans la préparation Y.

### Exemple 108 : 1-(R)-1-(Benzhydrylaminocarbonylcyclopentanecarboxamido)-4-guanidinobutyl boronate de (+)α-pinanediol, benzène sulfonate

Le produit attendu est obtenu selon le procédé décrit dans l'exemple 2 à partir du composé décrit dans l'exemple 107.

### Exemple 109 : Acide 1-(R)-1-(benzhydrylaminocarbonylcyclopentanecarboxamido)-4-guanidino boronique, benzène sulfonate

Le produit attendu est obtenu selon le procédé décrit dans l'exemple 22 à partir du composé décrit dans l'exemple 108.

### Exemple 110 : 1-(R)-[1-(2,2-Diphényléthylaminocarbonyl)cyclopentanecarboxamido]-4-aminobutyl boronate de (+)α-pinanediol, benzène sulfonate

Le produit attendu est obtenu selon le procédé décrit dans l'exemple 20 en utilisant comme produit de départ le composé décrit dans la préparation Z.

### Exemple 111 1-(R)-[1-(2,2-Diphényléthylaminocarbonyl)cyclopentanecarboxamido]-4-guanidinobutyl boronate de (+)α-pinanediol, benzène sulfonate

Le produit attendu est obtenu selon le procédé décrit dans l'exemple 2 à partir du composé décrit dans l'exemple 110.

### Exemple 112 : Acide 1-(R)-[1-(2,2-diphényléthylaminocarbonyl)-cyclopentane carboxamido]-4-guanidinobutyl boronique, benzène sulfonate

Le produit attendu est obtenu selon le procédé décrit dans l'exemple 22 à partir du composé décrit dans l'exemple 111.

### Exemple 113 : 1-(R)-[1-(Naphtalen-2-yl-méthylaminocarbonyl)cyclopentanecarboxamido]-4-aminobutyl boronate de (+)α-pinanediol, benzène sulfonate

Le produit attendu est obtenu selon le procédé décrit dans l'exemple 20 en utilisant comme produit de départ le composé décrit dans la préparation AA.

### Exemple 114 : 1-(R)-[1-Naphthalen-2-yl-méthylaminocarbonyl)cyclopentane carboxamido]-4-guanidinobutyl boronate de (+)α-pinanediol, benzène sulfonate

Le produit attendu est obtenu selon le procédé décrit dans l'exemple 2 à partir du composé décrit dans l'exemple 113.

### Exemple 115 : Acide 1-(R)-[1-(naphtalen-2-yl-méthylaminocarbonyl)cyclopentane carboxamido]-4-guanidinobutyl boronique, benzène sulfonate

Le produit attendu est obtenu selon le procédé décrit dans l'exemple 22 à partir du composé décrit dans l'exemple 114.

### Exemple 116 : 1-(R)-{1-[2-(Naphtalen-2-yl)-éthylaminocarbonyl]cyclopentane carboxamido}-4-aminobutyl boronate de (+)α-pinanediol, benzene sulfonate

Le produit atttendu est obtenu selon le procédé décrit dans l'exemple 20, en utilisant comme produit de départ le composé décrit dans la préparation AB.

### Exemple 117 : 1-(R)-{1-[2-(Naphthalen-2-yl)-éthylaminocarbonyl]cyclopentane carboxamido}-4-guanidinobutyl boronate de (+)α-pinanediol. benzène sulfonate

Le produit attendu est obtenu selon le procédé décrit dans l'exemple 2 à partir du composé décrit dans l'exemple 116.

### Exemple 118 : Acide 1-(R)-{1-[2-(naphtalen-2-yl)-éthylaminocarbonyl]cyclopentane carboxamido}-4-guanidinobutyl boronique, benzène sulfonate

Le produit attendu est obtenu selon le procédé décrit dans l'exemple 22 à partir du composé décrit dans l'exemple 117.

### Exemple 119 : 1-(R)-[1-(1,1-Diméthyl-2-phényléthylaminocarbonyl)cyclopentane carboxamido]-4-aminobutyl boronate de (+)α-pinanediol, benzène sulfonate

Le produit attendu est obtenu selon le procédé décrit dans l'exemple 20 en utilisant comme produit de départ le composé décrit dans la préparation AC.

### Exemple 120 : 1-(R)-[1-(1,1-Diméthyl-2-phényléthylaminocarbonyl)cyclopentane carboxamido]-4-guanidinobutyl boronate de (+)α-pinanediol, benzène sulfonate

Le produit attendu est obtenu selon le procédé décrit dans l'exemple 2 à partir du composé décrit dans l'exemple 119.

### Exemple 121 : Acide 1-(R)-[1-(1,1-diméthyl-2-phényléthylaminocarbonyl)cyclopentane carboxamido]-4-guanidinobutyl boronique, benzène sulfonate

Le produit attendu est obtenu selon le procédé décrit dans l'exemple 22 à partir du composé décrit dans l'exemple 120.

### Exemple 122 : 1-(R)-[1-(3-Phénylpropylaminocarbonyl)cyclopentanecarboxamido]-4-aminobutyl boronate de (+) α-pinanediol, benzène sulfonate

Le produit attendu est obtenu selon le procédé décrit dans l'exemple 20 en utilisant comme produit de départ le composé décrit dans la préparation AD.

### Exemple 123 : 1-(R)-[1-(3-Phénylpropylaminocarbonyl)cyclopentanecarboxamido]-4-guanidinobutyl boronate de (+) α-pinanediol, benzène sulfonate

Le produit attendu est obtenu selon le procédé décrit dans l'exemple 2 à partir du composé décrit dans l'exemple 122.

### Exemple 124 : Acide 1-(R)-[1-(3-phénylpropylaminocarbonyl)cyclopentane carboxamido]-4-guanidinobutyl boronique, benzène sulfonate

Le produit attendu est obtenu à selon le procédé décrit dans l'exemple 22 à partir du composé décrit dans l'exemple 123.

### Exemple 125 : 1-(R)-[1-(2-Benzènesulfonylaminoéthylaminocarbonyl)cyclopentane carboxamido]-4-aminobutyl boronate de (+) α-pinanediol. benzène sulfonate

Le produit attendu est obtenu selon le procédé décrit dans l'exemple 20 en utilisant comme produit de départ le composé décrit dans la préparation AE.

### Exemple 126 : 1-(R)-[1-(2-Benzènesulfonylaminoéthylaminocarbonyl)cyclopentane carboxamido]-4-guanidinobutyl boronate de (+) α-pinanediol, benzène sulfonate

Le produit attendu est obtenu selon le procédé décrit dans l'exemple 2 à partir du composé décrit dans l'exemple 125.

### Exemple 127 : Acide 1-(R)-[1-(2-benzènesulfonylaminoéthylaminocarbonyl) cyclopentanecarboxamido]-4-guanidinobutyl boronique, benzène sulfonate

Le produit attendu est obtenu selon le procédé décrit dans l'exemple 22 à partir du composé décrit dans l'exemple 126.

### Exemple 128 : 1-(R)-{1-[4-(4-Méthoxybicyclo[2,2,2]oct-1-yl)benzylaminocarbonyl] cyclopentanecarboxamido}-4-aminobutyl boronate de (+) α-pinanediol, benzène sulfonate

Le produit attendu est obtenu selon le procédé décrit dans l'exemple 20 en utilisant comme produit de départ le composé décrit dans la préparation AF.

### Exemple 129 : 1-(R)-{1-[4-(4-Méthoxybicyclo[2,2,2]-oct-1-yl)benzylaminocarbonyl] cyclopentanecarboxamido}-4-guanidinobutyl boronate de (+) α-pinanediol, benzène sulfonate

Le produit attendu est obtenu selon le procédé décrit dans l'exemple 2 à partir du composé décrit dans l'exemple 128.

### Exemple 130 : Acide 1-(R)-{1-[4-(4-méthoxybicyclo[2.2.2]-oct-1-yl)benzylamino carbonyl]cyclopentanecarboxamido}-4-guanidinobutyl boronique, benzène sulfonate

Le produit attendu est obtenu selon le procédé décrit dans l'exemple 22 à partir du composé décrit dans l'exemple 129.

### Exemple 131 : 1(R)-[1-(Indan-2-yl-aminocarbonyl)cyclopentanecarboxamido]-4-aminobutyl, boronate de (+) α-pinanediol, benzène sulfonate

Le produit attendu est obtenu selon le procédé décrit dans l'exemple 20 en utilisant comme produit de départ le composé décrit dans la préparation AG.

### Exemple 132 : 1-(R)-[1-(Indan-2-yl-aminocarbonyl)cyclopentanecarboxamido]-4-guanidinobutyl, boronate de (+) α-pinanediol, benzène sulfonate

Le produit attendu est obtenu selon le procédé décrit dans l'exemple 2 à partir du composé décrit dans l'exemple 131.

### Exemple 133 : Acide 1-(R)-[1-(indan-2-yl-aminocarbonyl)cyclopentanecarboxamido]-4-guanidinobutyl boronique, benzène sulfonate

Le produit attendu est obtenu selon le procédé décrit dans l'exemple 22 à partir du composé décrit dans l'exemple 132.

### Exemple 134 : 1-(R)-[1-(Indan-2-yl-méthylaminocarbonyl)cyclopentanecarboxamido]-4-aminobutyl boronate de (+) α-pinanediol, benzène sulfonate

Le produit attendu est obtenu selon le procédé décrit dans l'exemple 20 en utilisant comme produit de départ le composé décrit dans la préparation AH.

### Exemple 135 : 1-(R)-[1-(Indan-2-yl-méthylaminocarbonyl)cyclopentanecarboxamido]-4-guanidinobutyl boronate de (+) α-pinanediol, benzène sulfonate

Le produit attendu est obtenu selon le procédé décrit dans l'exemple 2 à partir du composé décrit dans l'exemple 134.

### Exemple 136 : Acide 1-(R)-[1-(indan-2-yl-méthylaminocarbonyl)cyclopentane carboxamido]-4-guanidinobutyl boronique, benzène sulfonate

Le produit attendu est obtenu selon le procédé décrit dans l'exemple 22 à partir du composé décrit dans l'exemple 135.

### Exemple 137 : 1-(R)-[1-(10,11-Dihydro-5-H-dibenzo[a,d]cyclohepten-5-yl-aminocarbonyl)cyclopentanecarboxamido]-4-aminobutyl boronate de (+) α-pinanediol, benzène sulfonate

Le produit attendu est obtenu selon le procédé décrit dans l'exemple 20 en utilisant comme produit de départ le composé décrit dans la préparation AI.

### Exemple 138 : 1-(R)-[1-(10,11-Dihydro-5-H-dibenzo[a,d]cyclohepten-5-yl-aminocarbonyl)cyclopentanecarboxamido]-4-guanidinobutyl boronate de (+) α-pinanediol, benzène sulfonate

Le produit attendu est obtenu selon le procédé décrit dans l'exemple 2 à partir du composé décrit dans l'exemple 137.

### Exemple 139 : Acide 1-(R)-[1-(10,11-dihydro-5-H-dibenzo[a,d]cyclohepten-5-yl-aminocarbonyl)cyclopentanecarboxamido]-4-guanidinobutyl boronique, benzène sulfonate

Le produit attendu est obtenu selon le procédé décrit dans l'exemple 22 à partir du composé décrit dans l'exemple 138.

### Exemple 140 : 1-(R)-[1-(6,11-Dihydro-6-méthyl-dibenzo[c,f][1,2]thiazepin-11-yl-aminocarbonyl)cyclopentane carboxamido]-4-aminobutyl boronate de (+) α-pinanediol, benzène sulfonate

Le produit attendu est obtenu selon le procédé décrit dans l'exemple 20 en utilisant comme produit de départ le composé décrit dans la préparation AJ.

### Exemple 141 : 1-(R)-[1-(6,11-Dihydro-6-méthyl-dibenzo[c,f] [1,2]thiazepin-11-yl-aminocarbonyl) cyclopentane carboxamido]-4-guanidinobutyl boronate de (+) α-pinanediol, benzène sulfonate

Le produit attendu est obtenu selon le procédé décrit dans l'exemple 2 à partir du composé décrit dans l'exemple 140.

### Exemple 142 : Acide 1-(R)-[1-(6,11-dihydro-6-méthyl-dibenzo[c,f][1,2]thiazepin-11-yl-aminocarbonyl)cyclopentane carboxamido]-4-guanidinobutyl boronique, benzène sulfonate

Le produit attendu est obtenu selon le procédé décrit dans l'exemple 22 à partir du composé décrit dans l'exemple 141.

### Exemple 143 : 1-(R)-[1-(1-Benzènesulfonyl-piperidin-4-yl-aminocarbonyl)-cyclopentane carboxamido]-4-aminobutyl boronate de (+) α-pinanediol, benzène sulfonate

Le produit attendu est obtenu selon le procédé décrit dans l'exemple 20 en utilisant comme produit de départ le composé décrit dans la préparation AK.

### Exemple 144 : 1-(R)-[1-Benzènesulfonyl-piperidin-4-yl-aminocarbonyl)-cyclopentane carboxamido]-4-guanidinobutyl boronate de (+) α-pinanediol, benzène sulfonate

Le produit attendu est obtenu selon le procédé décrit dans l'exemple 2 à partir du composé décrit dans l'exemple 143.

### Exemple 145 : Acide 1-(R)-[1-(1-benzènesulfonyl-piperidin-4-yl-aminocarbonyl)cyclopentane carboxamido]-4-guanidinobutyl boronique, benzène sulfonate

Le produit attendu est obtenu selon le procédé décrit dans l'exemple 22 à partir du composé décrit dans l'exemple 144.

### Exemple 146 : 1-(R)-{2-[1-(4-Méthoxybicyclo[2.2.2]oct-1-yl)-1-méthyl]éthylcarbonylamino}-4-aminobutyl boronate de (+)α-pinanediol, chlorhydrate

Le produit attendu est obtenu selon le procédé décrit dans l'exemple 1 en utilisant comme produit de départ le produit décrit dans la préparation AL.

### Exemple 147 : 1-(R)-{2-[1-(4-Méthoxybicyclo[2.2.2]oct-1-yl)-1-méthyl]éthylcarbonylamino}-4-guanidinobutyl boronate de (+)α-pinanediol, chlorhydrate

Le produit attendu est obtenu selon le procédé décrit dans l'exemple 2 à partir du composé de l'exemple 146.

### Exemple 148 : Acide 1-(R)-{2-[1-(4-Méthoxybicyclo[2.2.2]oct-1-yl)-1-méthyl]éthylcarbonyl-amino}-4-guanidinobutyl boronique, chlorhydrate

Le produit attendu est obtenu selon le procédé décrit dans l'exemple 3 à partir du composé décrit dans l'exemple 147.

### Etude pharmacologique des dérivés de l'invention

### Exemple 149 : Activite anti-coagulante, mesure de temps de thrombine et temps de céphaline activée chez l'homme

Afin d'évaluer l'activité anti-coagulante des composés de l'invention, le temps de thrombine (TT) et le temps de céphaline activée (TCA) ont été déterminés dans des échantillons de plasma humain. Un coagulomètre ST₄ (Diagnostica Stago, France) a été utilisé. Chez des volontaires sains, des prélèvements de sang veineux au pli de coude ont été réalisés dans une solution de citrate trisodique (0.109 M). Un plasma pauvre en plaquettes est obtenu par centrifugation des échantillons sanguins (3000 g, 15 minutes). Le TT est réalisé avec le réactif Thrombine Prest et le TCA avec le réactif Céphaline PTT Automate.
L'inhibiteur ou le solvant (10 µl) est additionné au plasma (90 µl), puis incubé 2 minutes à 37°C. 100 µl de Thrombine Prest (TT) ou de Céphaline PTT Automate (TCA) sont ajoutés en déclenchant le chronomètre.

Dans ces conditions, le TT est de l'ordre de 18 secondes, le TCA est de l'ordre de 12 secondes. L'activité d'un antagoniste est évaluée par sa capacité de prolonger le TT et le TCA par rapport au témoin. L'effet des inhibiteurs est exprimé par la concentration en µM qui multiplie de 2 le temps de coagulation (Ctt₂).

Les composés de l'invention ont produit des prolongations des temps de coagulation très importantes et les Ctt₂ sont exemplifiés dans le tableau ci-dessous :

| Produits | TT Ctt₂ (µM) | TCA Ctt₂ (µM) |
|---|---|---|
| Ex. 3 | 0.32 | 1.25 |
| Ex. 22 | 0.30 | 1.58 |
| Ex. 6 | 0.75 | 3.13 |
| Ex. 12 | 0.18 | 0.78 |
| Ex. 83 | 0.13 | 0.56 |
| Ex. 51 | 0.15 | 0.84 |
| Ex. 103 | 0.29 | 1.67 |
| Ex. 94 | 0.32 | 1.77 |
| Ex. 69 | 0.17 | 0.56 |

### Exemple 150 : Inhibition de la thrombine et de sérines protéases de la fibrinolyse

Pour évaluer in vitro l'activité inhibitrice des produits de l'invention sur la thrombine humaine (Sigma®, activité spécifique 3230 UNIH/mg), le fibrinogène humain purifié (4 mM, Stago®) (Fg) a été ajouté à une quantité donnée de thrombine (0.7 nM) préalablement incubée avec ou sans l'inhibiteur à tester (20°C, 10 minutes). (UNIH signifie unité de l'Institut National de la Santé).

Pour évaluer in vitro la sélectivité de ces produits vis-à-vis de différentes sérines protéases de la fibrinolyse, le même protocole a été appliqué à la plasmine humaine purifiée (2 nM, Stago®), à l'activateur tissulaire du plasminogène (t-PA) (2 nM, Calbiochem®) et à l'urokinase (u-PA) humaine purifié (2 nM, Sigma®), en utilisant pour substrats différents peptides paranitroanilidés : <Glu-Phe-Lys-pNA (0.50 mM, S 2403, Kabi®), H-D-Ile-Pro-Arg-pNA (0.48 mM, S 2288, Kabi®), <Glu-Gly-Arg-pNA (0.56 mM, S 2444, Kabi®).

Inhibiteurs, enzymes et substrats sont dilués dans le même tampon (tampon phosphate 0.01 mM, pH 7.4, contenant 0.12 M de chlorure de sodium et 0.05 % de sérum albumine bovine) puis distribués dans une microplaque en polystyrène sous un volume de 50 µl.

La fibrine formée par le thrombine ou par le paranitroanilide libéré par l'action de la sérine protéase est mesurée spectrophotométriquement à 405 nm après 15 à 30 minutes de réaction à 20°C.

Le tableau ci-dessous donne la concentration des composés en nM inhibant 50 % de l'activité enzymatique (CI₅₀) par rapport au contrôle sans produit. Les résultats obtenus démontrent que les composés de l'invention sont des inhibiteurs puissants de la thrombine humaine vis-à-vis du fibrinogène humain. Les composés possèdent une sélectivité très importante vis-à-vis des sérines protéases de la fibrinolyse.

Le tableau ci-dessous exemplifie les résultats obtenus pour les composés de l'invention :

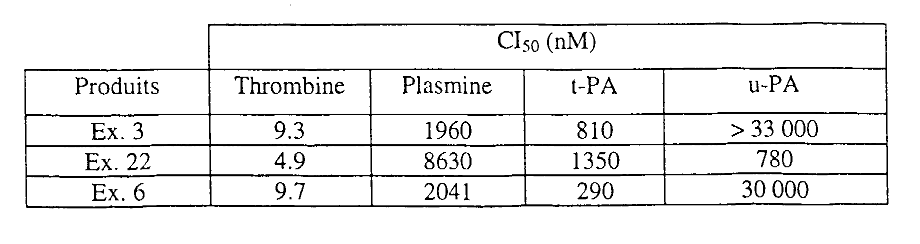

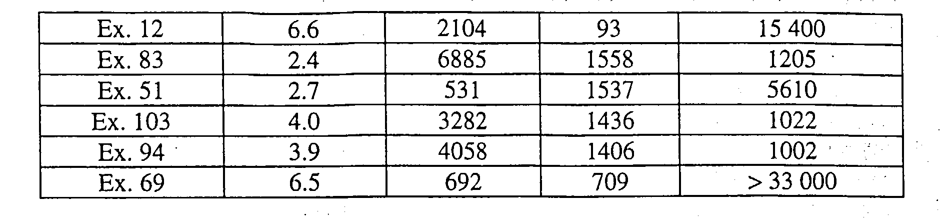

### Exemple 151 : Activité anti-coagulante et thrombopénique après administration per or chez le chien

Des chiens mâles ou femelles de 11-28 kg sont traités par voie orale avec les produits de l'invention (5 mg/kg) ou avec la substance de référence, le DUP 714 à 2,5 mg/kg. Les temps de coagulation (TT, TCA) sont déterminés dans des échantillons de plasma des chiens 10 min avant et 30 min, 1 heure, 2 heures, 4 heures et 6 heures après l'administration des produits. Les mesures des temps de coagulation sont effectuées comme décrits dans l'exemple 149. Le nombre de plaquettes est également déterminé dans chaque échantillon à l'aide d'un compteur T450 (Coultronics).

Dans les conditions de nos expériences, le nombre de plaquettes avant le traitement est de l'ordre de 300 000 plaquettes/µl ; le TT est de l'ordre de 19 secondes et le TCA de l'ordre de 18 secondes.

La substance de référence utilisée à la dose de 2,5 mg/kg baisse le nombre de plaquettes de façon importante et réversible ; à 1 heure après son administration, le nombre de plaquettes baisse de 55 ± 14 % (tableau ci-dessous). De tels thrombopénies ne sont pas observées avec les substances de l'invention utilisées à des doses plus fortes que le DUP 714, sauf l'exemple 12 :

| Thrombopénies observées 1 heure après administration p.o. chez le chien | |
|---|---|
| **Produits** | **%** |
| DUP 714 (2,5 mg/kg) | 55 % |
| Ex. 3 | 0 % |
| Ex. 22 | 0 % |
| Ex. 6 | 0 % |
| Ex. 12 | 40 % |
| Ex. 83 | 0 |
| Ex. 51 | 0 |
| Ex. 103 | 0 |
| Ex. 94 | 0 |
| Ex. 69 | 0 |

Le DUP 714 et les substances de l'invention augmentent de façon importante les TT et les TCA chez les animaux. Le tableau B résume les résultats obtenus. Les résultats sont montrés 1 heure et 4 heures après l'administration p.o. de 2,5 mg/kg du DUP 714 et de 5 mg/kg des substances de l'invention. Les valeurs montrent le nombre de fois que le temps initial est augmenté.

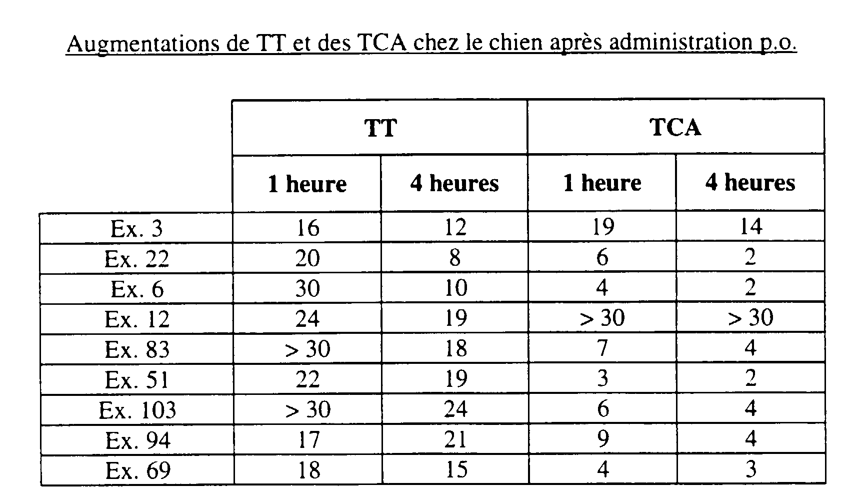

### Exemple 152 : Composition pharmaceutique

| Formule de préparation pour 1000 comprimés dosés à 10 mg : | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I): 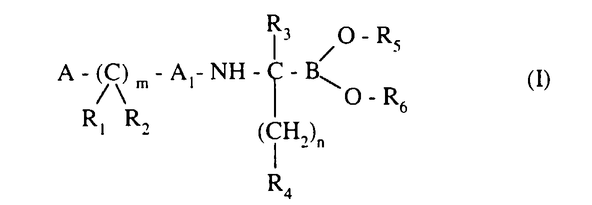 dans laquelle :
R₁, R₂, identiques ou différents, représentent un atome d'hydrogène, ou un groupement alkyle (C₁-C₆) linéaire ou ramifié ou bien R₁ et R₂ forment avec l'atome de carbone qui les porte un groupement cycloalkyle (C₃-C₈),
R₃ représente un atome d'hydrogène ou un groupement alkyle (C₁-C₆) linéaire ou ramifié, un groupement phényle éventuellement substitué ou un groupement benzyle éventuellement substitué,
R₄ représente :
- un groupement amino substitué éventuellement par un ou plusieurs groupements, identiques ou différents, alkyle (C₁-C₆) linéaire ou ramifié, benzyle éventuellement substitué, aryle ou hétérocyclique,
- un groupement amidino substitué éventuellement par un ou plusieurs groupements, identiques ou différents, alkyle (C₁-C₆) linéaire ou ramifié, benzyle éventuellement substitué, aryle ou hétérocyclique,
- un groupement guanidino substitué éventuellement par un groupement alkyle (C₁-C₆) linéaire ou ramifié, benzyle éventuellement substitué, aryle ou hétérocyclique,
- isothiouréïdo substitué éventuellement par un groupement alkyle (C₁-C₆) linéaire ou ramifié, benzyle éventuellement substitué, aryle ou hétérocyclique,
- iminométhylamino éventuellement substitué par un groupement alkyle (C₁-C₆) linéaire ou ramifié,
- mercapto substitué par un groupement hétérocyclique,
- ou, un groupement hétérocyclique,
R₅, R₆ réprésentent chacun un atome d'hydrogène ou un groupement alkyle (C₁-C₆) linéaire ou ramifié,
ou 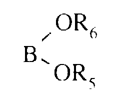 forme un ester boronique de pinanediol,
m représente un entier tel que 0 ≤ m ≤ 6,
n représente un entier tel que 1 ≤ n ≤ 6,
A représente l'un quelconque des groupements suivants :
* un groupement hicycloalkyle (C₅-C₁₀) phényle substitué éventuellement par un ou plusieurs, identiques ou différents, atomes d'halogène ou groupements alkyle (C₁-C₆) linéaire ou ramifié, alkoxy (C₁-C₆) linéaire ou ramifié, hydroxy ou amino (éventuellement substitué par un ou deux groupements, identiques ou différents, alkyle (C₁-C₆) linéaire ou ramifié, alkylsulfonyle (C₁-C₆) linéaire ou ramifié ou arylsulfonyle),
* ou, un groupement de formule : 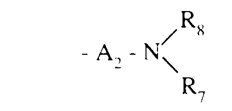 à la condition que, dans ce cas, m soit différent de zéro,
dans laquelle
R₇, R₈, identiques ou différents, représentent un atome d'hydrogène, un groupement alkyle (C₁-C₆) linéaire ou ramifié (éventuellement substitué par un ou plusieurs groupements aryle, hétérocyclique, arylsulonylamino, bicycloalkyle (C₅-C₁₀) phényle éventuellement substitué par un ou plusieurs groupements tels que définis plus haut ou indanyle), un groupement alkylsulfonyle (C₁-C₆) linéaire ou ramifié, un groupement arylsulfonyle, un groupement aryle, un groupement hétérocyclique ou un groupement bicycloalkyle (C₅-C₁₀) phényle (éventuellement substitué par un ou plusieurs groupements tels que définis plus haut), un groupement indanyle ou l'un quelconque des groupements : 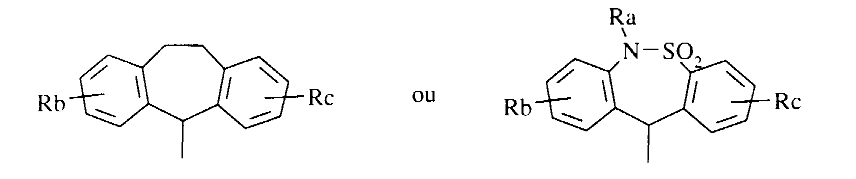 dans lesquels
Ra représente un groupement alkyle (C₁-C₆) linéaire ou ramifié ou phényle éventuellement substitué,
Rb, Rc, identiques ou différents, représentent un atome d'hydrogène, d'halogène, ou un groupement alkyle (C₁-C₆) linéaire ou ramifié, alkoxy (C₁-C₆) linéaire ou ramifié, hydroxy, amino substitué ou non, ou trihalogénométhyle,
A₂ représente -CO- ou -CS-,
A₁ représente -CO-, -CS- ou -SO₂-,
ses isomères ainsi que ses sels d'addition à un acide ou à une base pharmaceutiquement acceptable.

2. Composé de formule (I) selon la revendication 1 tels que A₁ représente -CO-.

3. Composé de formule (I) selon la revendication 1 tels que R₃ représente un hydrogène.

4. Composé de formule (I) selon la revendication 1 tel que R₄ représente un groupement guanidino substitué éventuellement par un groupement alkyle (C₁-C₆) linéaire ou ramifié, benzyle éventuellement substitué, aryle ou hétérocyclique.

5. Composé de formule (I) selon la revendication 4 tel que R₄ représente un groupement guanidino.

6. Composé de formule (I) selon la revendication 1 tel que A représente un groupement bicycloalkyle (C₅-C₁₀) phényle substitué éventuellement par un ou plusieurs, identiques ou différents, atomes d'halogène ou groupements alkyle (C₁-C₆) linéaire ou ramifié, alkoxy (C₁-C₆) linéaire ou ramifié, hydroxy ou amino (éventuellement substitué par un ou deux groupements, identiques ou différents, alkyle (C₁-C₆) linéaire ou ramifié, alkylsulfonyle (C₁-C₆) linéaire ou ramifié ou arylsulfonyle).

7. Composé de formule (I) selon la revendication 6 tel que A représente un groupement (bicyclo[2.2.2]oct-1-yl)phényle éventuellement substitué.

8. Composé de formule (I) selon la revendication 6 tel que m est égal à 0.

9. Composé de formule (I) selon la revendication 1 tel que A représente

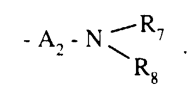

10. Composé de formule (I) selon la revendication 9 tel que A₂ représente -CO-, R₇ représente un atome d'hydrogène et R₈ représente un groupement alkyle (C₁-C₆) linéaire ou ramifié, substitué par un ou plusieurs groupements aryle.

11. Composé de formule (I) selon la revendication 9 tel que m est égal à 1.

12. Composé de formule (I) selon la revendication 9 tels que R₁, R₂ forment avec l'atome de carbone qui les porte un groupement cycloalkyle (C₃-C₈).

13. Composé de formule (I) selon la revendication 12 tel que R₁, R₂ forment avec l'atome de carbone qui les porte un groupement cyclopentyle.

14. Composé de formule (I) selon la revendication 1 tels que R₅, R₆ représentent chacun un atome d'hydrogène.

15. Composé de formule (I) selon la revendication 1 qui est l'acide 1-(R)-[4-(4-méthoxybicyclo[2.2.2]oct-1-yl)benzoylamino]-4-guanidinobutyl boronique, ainsi que ses sels d'addition à un acide ou à une base pharmaceutiquement acceptable.

16. Composé de formule (I) selon la revendication 1 qui est l'acide 1-(R)-(1-benzylaminocarbonylcyclopentanecarboxamido)-4-guanidinobutyl boronique, ainsi que ses sels d'addition à un acide ou à une base pharmaceutiquement acceptable.

17. Composé de formule (I) selon la revendication 1 qui est l'acide 1-(R)-[4-(4-hydroxybicyclo[2.2.2]oct-1-yl)benzoylamino]-4-guanidinobutyl boronique, ainsi que ses sels d'addition à un acide ou à une base pharmaceutiquement acceptable.

18. Composé de formule (I) selon la revendication 1 qui est l'acide 1-(R)-(2-benzylaminocarbonyl-2-méthylpropionamido)-4-guanidinobutyl boronique, ainsi que ses sels d'addition à un acide ou à une base pharmaceutiquement acceptable.

19. Composé de formule (I) selon la revendication 1 qui est l'acide 1-(R)-[3-(4-méthoxybicyclo[2.2.2]oct-1-yl)benzoylamino]-4-guanidinobutylboronique, ainsi que ses sels d'addition à un acide ou à une base pharmaceutiquement acceptable.

20. Composé de formule (I) selon la revendication 1 qui est l'acide 1-(R)-[3-(4-hydroxybicyclo[2.2.2]oct-1-yl)benzoylamino]-4-guanidinobutyl boronique, ainsi que ses sels d'addition à un acide ou à une base pharmaceutiquement acceptable.

21. Composé de formule (I) selon la revendication 1 qui est l'acide 1-(R)-(1-phénéthylaminocarbonylcyclopentanecarboxamido)-4-guanidinobutyl boronique, ainsi que ses sels d'addition à un acide ou à une base pharmaceutiquement acceptable.

22. Composé de formule (I) selon la revendication 1 qui est l'acide 1-(R)-[1-(3,4-diméthoxyphénéthylaminocarbonyl)cyclopentane carboxamido]-4-guanidinobutyl boronique, ainsi que ses sels d'addition à un acide ou à une base pharmaceutiquement acceptable.

23. Composé de formule (I) selon la revendication 1 qui est l'acide 1-(R)-1-(benzhydrylaminocarbonylcyclopentanecarboxamido)-4-guanidinobutyl boronique, ainsi que ses sels d'addition à un acide ou à une base pharmaceutiquement acceptable.

24. Composé de formule (I) selon la revendication 1 qui est l'acide 1-(R)-[1-(10,11-dihydro-5-H-dibenzo[a,d]cyclohepten-5-yl-aminocarbonyl)cyclopentanecarboxamido]-4-guanidinobutyl boronique, ainsi que ses sels d'addition à un acide ou à une base pharmaceutiquement acceptable.

25. Procédé de préparation des composés de formule (I) caractérisé en ce que l'on met en réaction un composé de formule (II) : 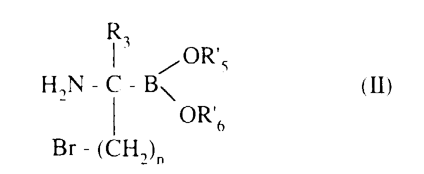 dans laquelle R₃ et n ont la même signification que dans la formule (I), R'₅ et R'₆ représentent chacun un groupement alkyle (C₁-C₆) linéaire ou ramifié ou 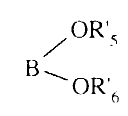 forme un ester boronique de pinanediol,
avec un composé de formule (III) : 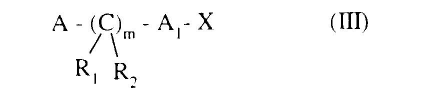 dans laquelle A, A₁, R₁, R₂ et m ont la même signification que dans la formule (I), X représente un atome de chlore ou un groupement hydroxy,
pour conduire au composé de formule (IV) : 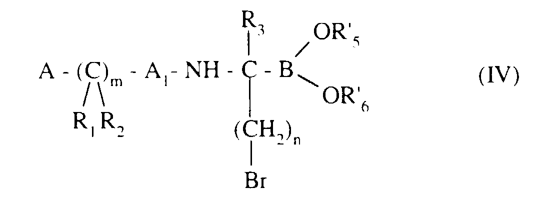 dans laquelle A, A₁, R₁, R₂, R₃, R'₅, R'₆, m et n ont la même signification que précédemment, composé de formule (IV) qui peut être transformé selon la nature du groupement R₄ que l'on souhaite obtenir :
- **soit**, en dérivé cyano correspondant par action du cyanure de cuivre puis, réaction en milieu alcoolique en présence d'acide suivi de l'action de l'ammoniaque, en dérivé amidino correspondant de (I/a), cas particulier des composés de formule (I): 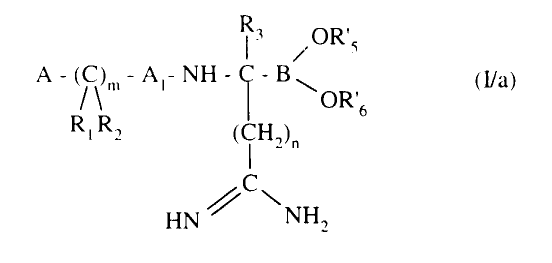 dans laquelle A, A₁, R₁, R₂, R₃, R'₅, R'₆, m et n ont la même signification que précédemment, dont on substitue, si on le désire, la fonction amidino,
- **soit**, en dérivé azido correspondant par action de l'azoture de sodium, puis par hydrogénation catalytique en dérivé amino correspondant de formule (I/b), cas particulier des composés de formule (I): 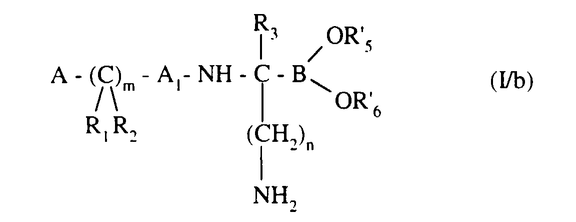 dans laquelle A, A₁, R₁, R₂, R₃, R'₅, R'₆, m et n ont la même signification que précédemment, composé de formule (I/b) dont on substitue, si on le désire, la fonction amine,
et dont on transforme, si on le désire, le groupement amino :
- en *groupement guanidino* par réaction avec du cyanamide,
pour conduire au composé de formule (I/c), cas particulier des composés de formule (I): 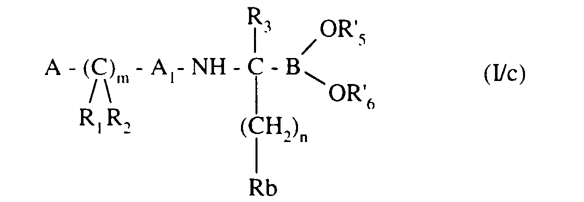 dans laquelle A, A₁, R₁, R₂, R₃, R'₅, R'₆, m et n ont la même signification que précédemment et Rb représente un groupement guanidino, dont on substitue, si on le désire, la fonction guanidino,
- ou, en *groupement iminométhylamino* par réaction avec du formimidate d'éthyle, pour conduire au composé de formule (I/d): 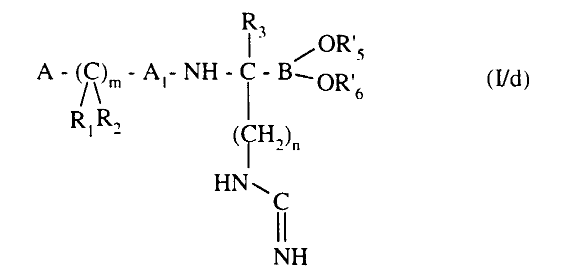 dans laquelle A, A₁, R₁, R₂, R₃, R'₅, R'₆, m et n ont la même signification que précédemment, dont on substitue, si on le désire, la fonction aminido,
- **soit**, par réaction avec une thiourée éventuellement substituée,
pour conduire au composé de formule (I/e), cas particulier des composés de formule (I): 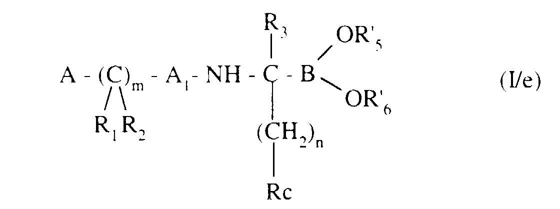 dans laquelle A, A₁, R₁, R₂, R₃, R'₅, R'₆, m et n ont la même signification que précédemment, et Rc représente un groupement isothiouréïdo éventuellement substitué,
- **soit**, par réaction avec un hétérocycle, convenablement protégé, une amine substituée par un hétérocycle convenablement protégé ou un thiol substitué par un hétérocycle convenablement protégé,
pour conduire, après déprotection, au composé de formule (I/f), cas particulier des composés de formule (I): 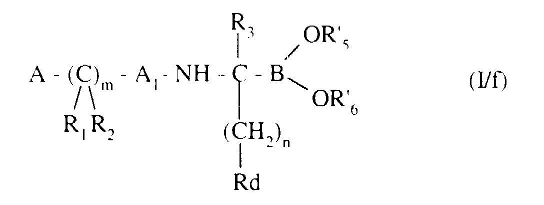 dans laquelle A, A₁, R₁, R₂, R₃, R'₅, R'₆, m et n ont la même signification que précédemment, et Rd représente un hétérocycle, un groupement amino substitué par un hétérocycle ou un groupement mercapto substitué par un hétérocycle,
composés de formule (I/a), (I/b), (I/c), (I/d), (I/e) ou (I/f), que l'on transforme, si on le souhaite, à l'aide de trichlorure de bore ou d'acide phénylboronique, en acide boronique correspondant de formule (I/g): 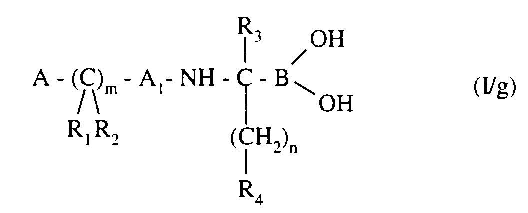 dans laquelle A, A₁, R₁, R₂, R₃, R₄, m et n ont la mjme signification que dans la formule (I),
composés de formule (I/a) à (I/g) :
- qui peuvent être, le cas échéant, purifiés selon une technique classique de purification,
- dont on sépare, le cas échéant, les isomères selon une technique classique de séparation,
- que l'on transforme, si on le souhaite, en ses sels d'addition à un acide ou à une base pharmaceutiquement acceptable.

26. Compositions pharmaceutiques contenant comme principe actif au moins un composé selon l'une quelconques revendications 1 à 24, seul ou en combinaison avec un ou plusieurs véhicules inertes, non toxiques pharmaceutiquement acceptables.

27. Compositions pharmaceutiques selon la revendication 26 utiles en tant qu'inhibiteurs de trypsine-like serine proteases.

28. Compositions pharmaceutiques selon la revendication 27 utiles en tant qu'inhibiteurs de thrombine.

## Patentansprüche

1. Verbindung der Formel (I): 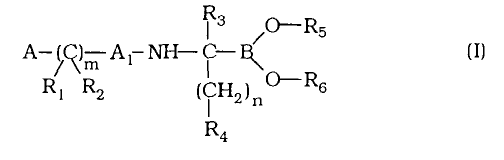 in der:
R₁ und R₂, die gleich oder verschieden sind, ein Wasserstoffatom oder eine geradkettige oder verzweigte (C₁-C₆)-Alkylgruppe bedeuten oder R₁ und R₂ mit dem sie tragenden Kohlenstoffatom eine (C₃-C₈)-Cycloalkylgruppe bilden,
R₃ ein Wasserstoffatom oder eine geradkettige oder verzweigte (C₁-C₆)-Alkylgruppe, eine gegebenenfalls substituierte Phenylgruppe oder eine gegebenenfalls substituierte Benzylgruppe darstellt,
R₄:
- eine Aminogruppe, die gegebenenfalls durch eine oder mehrere gleichartige oder verschiedene, geradkettige oder verzweigte (C₁-C₆) -Alkylgruppen, gegebenenfalls substituierte Benzylgruppen, Arylgruppen oder heterocyclische Gruppen substituiert ist,
- eine Amidinogruppe, die gegebenenfalls durch eine oder mehrere gleichartige oder verschiedene, geradkettige oder verzweigte (C₁-C₆)-Alkylgruppen. gegebenenfalls substituierte Benzylgruppen, Arylgruppen oder heterocyclische Gruppen substituiert ist,
- eine Guanidinogruppe, die gegebenenfalls durch eine geradkettige oder verzweigte (C₁-C₆)-Alkylgruppe, eine gegebenenfalls substituierte Benzylgruppe, eine Arylgruppe oder eine heterocyclische Gruppe substituiert ist,
- eine Isothioureidogruppe, die gegebenenfalls durch eine geradkettige oder verzweigte (C₁-C₆)-Alkylgruppe, eine gegebenenfalls substituierte Benzylgruppe, eine Arylgruppe oder eine heterocyclische Gruppe substituiert ist,
- eine Iminomethylaminogruppe, die gegebenenfalls durch eine geradkettige oder verzweigte (C₁-C₆)-Alkylgruppe substituiert ist,
- eine Mercaptogruppe, die durch eine heterocyclische Gruppe substituiert ist,
- oder eine heterocyclische Gruppe
darstellt,
R₅ und R₆ jeweils ein Wasserstoffatom oder eine geradkettige oder verzweigte (C₁-C₆)-Alkylgruppe bedeuten, oder 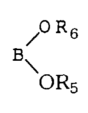 einen Boronsäureester von Pinandiol bildet,
m eine ganze Zahl von 0 ≤ m ≤ 6 darstellt,
n eine ganze Zahl von 1 ≤ n ≤ 6 darstellt,
A eine der folgenden Gruppen bedeutet:
* eine (C₅-C₁₀)-Bicycloalkyl-phenylgruppe, die gegebenenfalls substituiert ist durch ein oder mehrere, gleichartige oder verschiedene Halogenatome oder geradkettige oder verzweigte (C₁-C₆)-Alkylgruppen, geradkettige oder verzweigte (C₁-C₆)-Alkoxygruppen, Hydroxygruppen oder Aminogruppen (die gegebenenfalls durch eine oder zwei gleichartige oder verschiedene, geradkettige oder verzweigte (C₁-C₆)-Alkylgruppen, geradkettige oder verzweigte (C₁-C₆)-Alkylsulfonylgruppen oder Arylsulfonylgruppen substituiert ist),
* oder eine Gruppe der Formel: 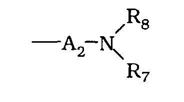 mit der Maßgabe, daß in diesem Fall m Null ist,
in der:
R₇ und R₈, die gleichartig oder verschieden sind, ein Wasserstoffatom, eine geradkettige oder verzweigte (C₁-C₆)-Alkylgruppe (die gegebenenfalls durch eine oder mehrere Arylgruppen, heterocyclische Gruppen, Arylsulfonylaminogruppen, gegebenenfalls durch eine oder mehrere Gruppen, wie sie oben definiert worden sind, substituierte (C₅-C₁₀)-Bicycloalkylphenylgruppen oder Indanylgruppen substituiert ist), eine geradkettige oder verzweigte (C₁-C₆)-Alkylsulfonylgruppe, eine Arylsulfonylgruppe, eine Arylgruppe, eine heterocyclische Gruppe oder eine (gegebenenfalls durch eine oder mehrere Gruppen, wie sie oben definierten worden sind, substituierte) (C₅-C₁₀)-Bicycloalkyl-phenylgruppe, eine Indanylgruppe oder eine der folgenden Gruppen bedeuten: 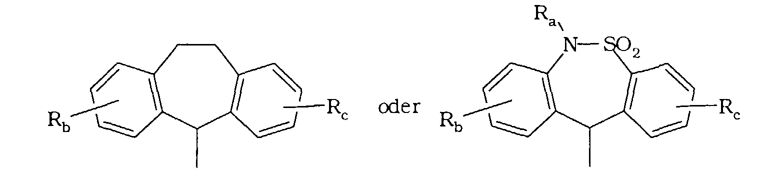 worin:
Rₐ eine geradkettige oder verzweigte (C₁-C₆)-Alkylgruppe oder eine gegebenenfalls substituierte Phenylgruppe darstellt und
R_{b} und R_{c}, die gleichartig oder verschieden sind, ein Wasserstoffatom, ein Halogenatom oder eine geradkettige oder verzweigte (C₁-C₆)-Alkylgruppe, eine geradkettige oder verzweigte (C₁-C₆)-Alkoxygruppe, eine Hydroxygruppe, eine gegebenenfalls substituierte Aminogruppe oder eine Trihalogenmethylgruppe bedeuten,
A₂ -CO- oder -CS- darstellt,
A₁ -CO-, -CS- oder -SO₂- darstellt,
deren Isomere sowie deren Additionssalze mit einer pharmazeutisch annehmbaren Säure oder Base.

2. Verbindung der Formel (I) nach Anspruch 1, worin A₁ -CO- bedeutet.

3. Verbindung der Formel (I) nach Anspruch 1, worin R₃ Wasserstoff bedeutet.

4. Verbindung der Formel (I) nach Anspruch 1, worin R₄ eine Guanidinogruppe bedeutet, die gegebenenfalls durch eine geradkettige oder verzweigte (C₁-C₆)-Alkylgruppe, eine gegebenenfalls substituierte Benzylgruppe, eine Arylgruppe oder eine heterocyclische Gruppe substituiert ist.

5. Verbindung der Formel (I) nach Anspruch 1, worin R₄ eine Guanidinogruppe darstellt.

6. Verbindung der Formel (I) nach Anspruch 1, worin A eine (C₅-C₁₀)-Bicycloalkyl-phenylgruppe darstellt, die gegebenenfalls durch ein oder mehrere gleichartige oder verschiedene Halogenatome oder geradkettige oder verzweigte (C₁-C₆)-Alkylgruppen, geradkettige oder verzweigte (C₁-C₆)-Alkoxygruppen, Hydroxygruppen oder (gegebenenfalls durch eine oder zwei gleichartige oder verschiedene, geradkettige oder verzweigte (C₁-C₆)-Alkylgruppen, geradkettige oder verzweigte (C₁-C₆)-Alkylsulfonylgruppen oder Arylsulfonylgruppen substituierte) Aminogruppen substituiert ist.

7. Verbindung der Formel (I) nach Anspruch 6, worin A eine gegebenenfalls substituierte (Bicyclo[2.2.2]oct-1-yl)-phenylgruppe bedeutet.

8. Verbindung der Formel (I) nach Anspruch 6, worin m den Wert 0 besitzt.

9. Verbindung der Formel (I) nach Anspruch 1, worin A eine Gruppe der Formel 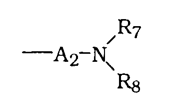 darstellt.

10. Verbindung der Formel (I) nach Anspruch 9, worin A₂ -CO-, R₇ ein Wasserstoffatom und R₈ eine geradkettige oder verzweigte (C₁-C₆)-Alkylgruppe, die durch eine oder mehrere Arylgruppen substituiert ist, bedeuten.

11. Verbindung der Formel (I) nach Anspruch 9, worin m den Wert 1 besitzt.

12. Verbindung der Formel (I) nach Anspruch 9, worin R₁ und R₂ zusammen mit dem sie tragenden Kohlenstoffatom eine (C₃-C₈)-Cycloalkylgruppe bilden.

13. Verbindung der Formel (I) nach Anspruch 12, worin R₁ und R₂ zusammen mit dem sie tragenden Kohlenstoffatom eine Cyclopentylgruppe bilden.

14. Verbindung der Formel (I) nach Anspruch 1, worin R₅ und R₆ jeweils ein Wasserstoffatom bedeuten.

15. Verbindung der Formel (I) nach Anspruch 1, nämlich 1-(R)-[4-(4-Methoxybicyclo[2.2.2]oct-1-yl)-benzoylamino]-4-guanidinobutyl-boronsäure sowie deren Additionssalze mit einer pharmazeutisch annehmbaren Säure oder Base.

16. Verbindung der Formel (I) nach Anspruch 1, nämlich 1-(R)-(1-Benzylaminocarbonylcyclopentancarboxamido)-4-guanidinobutyl-boronsäure sowie deren Additionssalze mit einer pharmazeutisch annehmbaren Säure oder Base.

17. Verbindung der Formel (I) nach Anspruch 1, nämlich 1-(R)-[4-(4-Hydroxybicyclo[2.2.2]oct-1-yl)-benzoylamino]-4-guanidinobutyl-boronsäure sowie deren Additionssalze mit einer pharmazeutisch annehmbaren Säure oder Base.

18. Verbindung der Formel (I) nach Anspruch 1, nämlich 1-(R)-(2-Benzylaminocarbonyl-2-methylpropionamido)-4-guanidinobutyl-boronsäure sowie deren Additionssalze mit einer pharmazeutisch annehmbaren Säure oder Base.

19. Verbindung der Formel (I) nach Anspruch 1, nämlich 1-(R)-[3-(4-Methoxybicyclo[2.2.2]oct-1-yl)-benzoylamino]-4-guanidinobutyl-boronsäure sowie deren Additionssalze mit einer pharmazeutisch annehmbaren Säure oder Base.

20. Verbindung der Formel (I) nach Anspruch 1, nämlich 1-(R)-[3-(4-Hydroxybicyclo[2.2.2]oct-1-yl)-benzoylamino]-4-guanidinobutyl-boronsäure sowie derenAdditionssalze mit einer pharmazeutisch annehmbaren Säure oder Base.

21. Verbindung der Formel (I) nach Anspruch 1, nämlich 1-(R)-(1-Phenethylaminocarbonylcyclopentancarboxamido)-4-guanidinobutyl-boronsäure sowie deren Additionssalze mit einer pharmazeutisch annehmbaren Säure oder Base.

22. Verbindung der Formel (I) nach Anspruch 1, nämlich 1-(R)-[1-(3,4-Dimethoxyphenethylaminocarbonyl)-cyclopentan-carboxamido]-4-guanidinobutyl-boronsäure sowie deren Additionssalze mit einer pharmazeutisch annehmbaren Säure oder Base.

23. Verbindung der Formel (I) nach Anspruch 1, nämlich 1-(R)-1-(Benzhydrylaminocarbonylcyclopentancarboxamido)-4-guanidinobutyl-boronsäure sowie deren Additionssalze mit einer pharmazeutisch annehmbaren Säure oder Base.

24. Verbindung der Formel (I) nach Anspruch 1, nämlich 1-(R)-[1-(10,11-Dihy-dro-5-H-dibenzo[a,d]cyclohepten-5-yl-aminocarbonyl)-cyclopentancarboxamido]4-guanidinobutyl-boronsäure sowie deren Additionssalze mit einer pharmazeutisch annehmbaren Säure oder Base.

25. Verfahren zur Herstellung der Verbindungen der Formel (I), dadurch gekennzeichnet, daß man eine Verbindung der Formel (II): 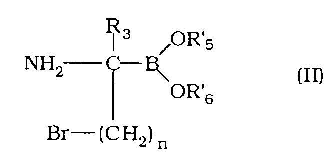 in der R₃ und n die bezüglich der Formel (I) angegebenen Bedeutungen besitzen und R'₅ und R'₆ jeweils eine geradkettige oder verzweigte (C₁-C₆)-Alkylgruppe darstellen oder die Gruppe 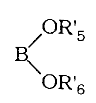 einen Boronsäureester von Pinandiol bildet,
mit einer Verbindung der Formel (III): 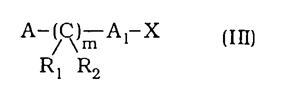 in der A, A1, R₁, R₂ und m die bezüglich der Formel (I) angegebenen Bedeutungen besitzen und
X ein Chloratom oder eine Hydroxygruppe darstellt,
umsetzt zur Bildung der Verbindung der Formel (IV): 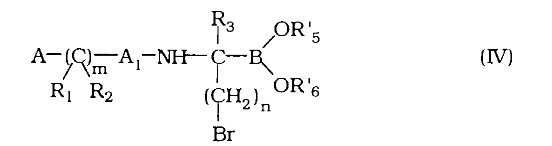 in der A, A₁, R₁, R₂, R₃, R'₅, R'₆, m und n die oben angegebenen Bedeutungen besitzen,
welche Verbindung der Formel (IV) in Abhängigkeit von der Art der Gruppe R₄, die man herzustellen wünscht, umgewandelt werden kann:
- **entweder** in das entsprechende Cyanoderivat durch Einwirkung von Kupfercyanid und dann Umsetzen in alkoholischem Medium in Gegenwart einer Säure gefolgt von der Einwirkung von Ammoniak zur Bildung des entsprechenden Amidinoderivats (I/a), einem Sonderfall der Verbindungen der Formel (I): 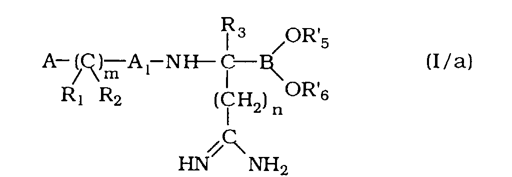 in der A, A₁, R₁, R₂, R₃, R'₅, R'₆, m und n die oben angegebenen Bedeutungen besitzen, deren Amidinofunktion man gewünschtenfalls substituiert,
- **oder** in das entsprechende Azidoderivat durch Einwirkung von Natriumazid und dann katalytische Hydrierung zu dem entsprechenden Aminoderivat der Formel (I/b), einem Sonderfall der Verbindungen der Formel (I): 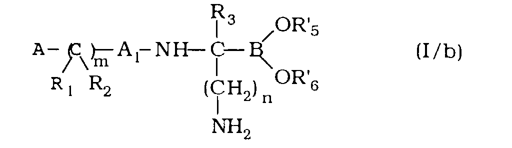 in der A, A₁, R₁, R₂, R₃, R'₅, R'₆, m und n die oben angegebenen Bedeutungen besitzen,
bei welcher Verbindung der Formel (I/b) man gewünschtenfalls die Aminofunktion substituiert,
und bei der man gewünschtenfalls die Aminogruppe umwandelt in:
- die *Guanidinogruppe* durch Umsetzen mit Cyanamid, zur Bildung der Verbindung der Formel (I/c), einem Sonderfall der Verbindungen der Formel (I): 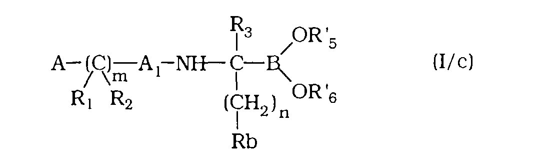 in der A, A₁, R₁, R₂, R₃, R'₅, R'₆, m und n cid oben angegebenen Bedeutungen besitzen und Rb eine Guanidinogruppe darstellt, wobei man gewünschtenfalls die Guanidinogruppe substituiert,
- oder in die *Iminomethylaminogruppe* durch Umsetzen mit Ethylformimidat zur Bildung der Verbindung der Formel (I/d): 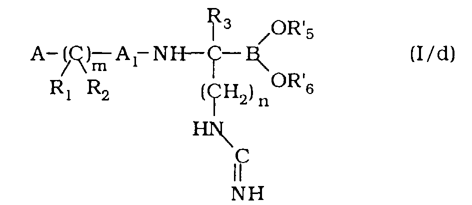 in der A, A₁, R₁, R₂, R₃, R'₅, R'₆, m und n die oben angegebenen Bedeutungen besitzen, bei der man gewünschtenfalls die Amidinofunktion substituiert,
- **oder** durch Umsetzen mit einem gegebenenfalls substituierten Thioharnstoff zur Bildung der Verbindung der Formel (I/e), einem Sonderfall der Verbindungen der Formel (I): 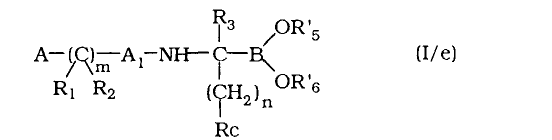 in der A, A₁, R₁, R₂, R₃, R'₅, R'₆, m und n die oben angegebenen Bedeutungen besitzen und Rc eine gegebenenfalls substituierte Isothioureidogruppe darstellt,
- **oder** durch Umsetzen mit einem in geeigneter Weise geschützten Heterocyclus, einem Amin, das durch einen geeignet geschützten Heterocyclus substituiert ist, oder einem Thiol, das durch einen geeignet geschützten Heterocyclus substituiert ist,
so daß man nach der Abspaltung der Schutzgruppe die Verbindung der Formel (I/f) erhält, einem Sonderfall der Verbindungen der Formel (I): 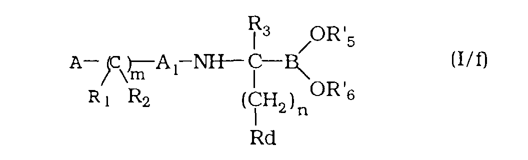 in der A, A₁, R₁, R₂, R₃, R'₅, R'₆, m und n die oben angegebenen Bedeutungen besitzen und Rd einen Heterocyclus, eine durch einen Heterocyclus substituierte Aminogruppe oder eine durch einen Heterocyclus substituierte Mercaptogruppe darstellt,
welche Verbindungen der Formeln (I/a), (I/b), (I/c), (I/d), (I/e) und (I/f) man gewünschtenfalls mit Hilfe von Bortrichlorid oder Phenylboronsäure in die entsprechende Boronsäure der Formel (I/g) umwandelt: 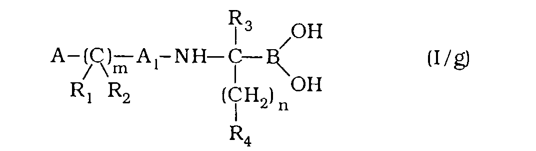 in der A, A₁, R₁, R₂, R₃, R₄, m und n die bezüglich der Formel (I) angegebenen Bedeutungen besitzen,
welche Verbindungen der Formeln (I/a) bis (I/g):
- gegebenenfalls mit Hilfe einer klassischen Reinigungsmethode gereinigt werden können,
- gegebenenfalls mit Hilfe einer klassischen Trennmethode in die Isomeren getrennt werden können,
- gewünschtenfalls mit Hilfe einer pharmazeutisch annehmbaren Säure oder Base in ihre Salze umgewandelt werden können.

26. Pharmazeutische Zubereitungen enthaltend als Wirkstoff mindestens eine Verbindung nach einem der Ansprüche 1 bis 24 allein oder in Kombination mit einem oder mehreren inerten, nichttoxischen, pharmazeutisch annehmbaren Trägermaterialien.

27. Pharmazeutische Zubereitungen nach Anspruch 26 zur Verwendung als Inhibitoren von Trypsin-artigen Serin-Proteasen.

28. Pharmazeutische Zubereitungen nach Anspruch 27 zur Verwendung als Thrombin-Inhibitoren.

## Claims

1. Compound of formula (I): 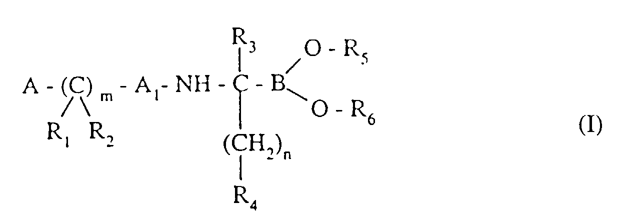 wherein:
R₁ and R₂, which may be identical or different, represent a hydrogen atom, or a linear or branched (C₁-C₆)alkyl group, or R₁ and R₂ form with the carbon atom carrying them a (C₃-C₈)cycloalkyl group,
R₃ represents a hydrogen atom or a linear or branched (C₁-C₆)alkyl group, an optionally substituted phenyl group or an optionally substituted benzyl group,
R₄ represents:
- an amino group optionally substituted by one or more, identical or different, groups : linear or branched (C₁-C₆)alkyl, optionally substituted benzyl, aryl or heterocyclic,
- an amidino group optionally substituted by one or more, identical or different, groups : linear or branched (C₁-C₆)alkyl, optionally substituted benzyl, aryl or heterocyclic,
- a guanidino group optionally substituted by a linear or branched (C₁-C₆)alkyl group, an optionally substituted benzyl group, an aryl group or by a heterocyclic group,
- isothioureido optionally substituted by a linear or branched (C₁-C₆)alkyl group, an optionally substituted benzyl group, an aryl group or by a heterocyclic group,
- iminomethylamino optionally substituted by a linear or branched (C₁-C₆)alkyl group,
- mercapto substituted by a heterocyclic group, or
- a heterocyclic group,
R₅ and R₆ each represents a hydrogen atom or a linear or branched (C₁-C₆)alkyl group, or 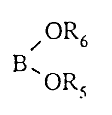 forms a pinanediol boronic ester,
m is an integer wherein 0 ≤ m ≤ 6,
n is an integer wherein 1 ≤ n ≤ 6,
A represents any one of the following groups:
* a (C₅-C₁₀)bicycloalkyl-phenyl group optionally substituted by one or more, identical or different, halogen atoms or groups : linear or branched (C₁-C₆)alkyl, linear or branched (C₁-C₆)alkoxy, hydroxy or amino (optionally substituted by one or two, identical or different, groups : linear or branched (C₁-C₆)alkyl, linear or branched (C₁-C₆)alkylsulphonyl or arylsulphonyl), or
* a group of the formula: 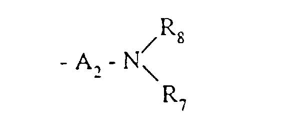 provided that, in that case, m is other than zero,
wherein:
R₇ and R₈, which may be identical or different, represent a hydrogen atom, a linear or branched (C₁-C₆)alkyl group (optionally substituted by one or more groups : aryl, heterocyclic, arylsulphonylamino, (C₅-C₁₀)bicycloalkyl-phenyl optionally substituted by one or more groups as defined above or indanyl), a linear or branched (C₁-C₆)alkylsulphonyl group, an arylsulphonyl group, an aryl group, a heterocyclic group or a (C₅-C₁₀)bicycloalkyl-phenyl group (optionally substituted by one or more groups as defined above), an indanyl group or either of the groups: 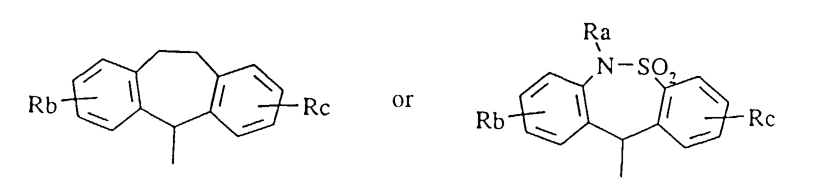 wherein:
Ra represents a linear or branched (C₁-C₆)alkyl group or an optionally substituted phenyl group,
Rb and Rc, which may be identical or different, represent a hydrogen atom, a halogen atom or a linear or branched (C₁-C₆)alkyl group, a linear or branched (C₁-C₆)alkoxy group, a hydroxy group, an unsubstituted or substituted amino group, or a trihalomethyl group,
A₂ represents -CO- or -CS-,
A₁ represents -CO-, -CS- or -SO₂-,
its isomers and addition salts thereof with a pharmaceutically acceptable acid or base.

2. Compound of formula (I) according to claim 1 wherein A₁ represents -CO-.

3. Compound of formula (I) according to claim 1 wherein R₃ represents hydrogen.

4. Compound of formula (I) according to claim 1 wherein R₄ represents a guanidino group optionally substituted by a linear or branched (C₁-C₆)alkyl group, an optionally substituted benzyl group, an aryl group or by a heterocyclic group.

5. Compound of formula (I) according to claim 4 wherein R₄ represents a guanidino group.

6. Compound of formula (I) according to claim 1 wherein A represents a (C₅-C₁₀)-bicycloalkyl-phenyl group optionally substituted by one or more, identical or different, halogen atoms or groups : linear or branched (C₁-C₆)alkyl, linear or branched (C₁-C₆)-alkoxy, hydroxy or amino (optionally substituted by one or two, identical or different, groups : linear or branched (C₁-C₆)alkyl, linear or branched (C₁-C₆)alkylsulphonyl or arylsulphonyl).

7. Compound of formula (I) according to claim 6 wherein A represents an optionally substituted (bicyclo[2.2.2]oct-1-yl)phenyl group.

8. Compound of formula (I) according to claim 6 wherein m is 0.

9. Compound of formula (I) according to claim 1 wherein A represents

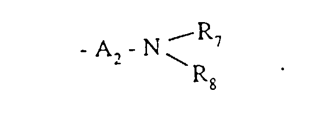

10. Compound of formula (I) according to claim 9 wherein A₂ represents -CO-, R₇ represents a hydrogen atom and R₈ represents a linear or branched (C₁-C₆)alkyl group substituted by one or more aryl groups.

11. Compound of formula (I) according to claim 9 wherein m is 1.

12. Compound of formula (I) according to claim 9 wherein R₁ and R₂ form with the carbon atom carrying them a (C₃-C₈)cycloalkyl group.

13. Compound of formula (I) according to claim 12 wherein R₁ and R₂ form with the carbon atom carrying them a cyclopentyl group.

14. Compound of formula (I) according to claim 1 wherein R₅ and R₆ each represents a hydrogen atom.

15. Compound of formula (I) according to claim 1 which is 1-(R)-[4-(4-methoxybicyclo[2.2.2]oct-1-yl)benzoylamino-4-guanidinobutylboronic acid, and its addition salts with a pharmaceutically acceptable acid or base.

16. Compound of formula (I) according to claim 1 which is 1-(R)-(1-benzylaminocarbonylcyclopentanecarboxamido)-4-guanidinobutylboronic acid, and its addition salts with a pharmaceutically acceptable acid or base.

17. Compound of formula (I) according to claim 1 which is 1-(R)-[4-(4-hydroxybicyclo[2.2.2]oct-1-yl)benzoylamino-4-guanidinobutylboronic acid, and its addition salts with a pharmaceutically acceptable acid or base.

18. Compound of formula (I) according to claim 1 which is 1-(R)-(2-benzylaminocarbonyl-2-methylpropionamido)-4-guanidinobutylboronic acid, and its addition salts with a pharmaceutically acceptable acid or base.

19. Compound of formula (I) according to claim 1 which is 1-(R)-[3-(4-methoxybicyclo[2.2.2]oct-1-yl)benzoylamino]-4-guanidinobutylboronic acid, and its addition salts with a pharmaceutically acceptable acid or base.

20. Compound of formula (I) according to claim 1 which is 1-(R)-[3-(4-hydroxybicyclo[2.2.2]oct-1-yl)benzoylamino]-4-guanidinobutylboronic acid, and its addition salts with a pharmaceutically acceptable acid or base.

21. Compound of formula (I) according to claim 1 which is 1-(R)-(1-phenethylaminocarbonylcyclopentanecarboxamido)-4-guanidinobutylboronic acid, and its addition salts with a pharmaceutically acceptable acid or base.

22. Compound of formula (1) according to claim 1 which is 1-(R)-[1-(3,4-dimethoxyphenethylaminocarbonyl)cyclopentanecarboxamido]-4-guanidinobutylboronic acid, and its addition salts with a pharmaceutically acceptable acid or base.

23. Compound of formula (1) according to claim 1 which is 1-(R)-1-(benzhydrylaminocarbonylcyclopentanecarboxamido)-4-guanidinobutylboronic acid, and its addition salts with a pharmaceutically acceptable acid or base.

24. Compound of formula (I) according to claim 1 which is 1-(R)-[1-(10,11-dihydro-5H-dibenzo[a,d]cyclohepten-5-yl-aminocarbonyl)cyclopentanecarboxamido]-4-guanidinobutylboronic acid, and its addition salts with a pharmaceutically acceptable acid or base.

25. Process for the preparation of compounds of formula (I), characterised in that a compound of formula (II): 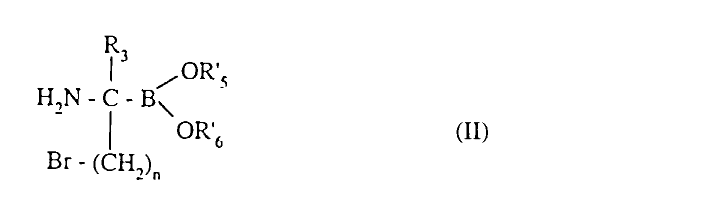 wherein R₃ and n are as defined for formula (I), R'₅ and R'₆ each represents a linear or branched (C₁-C₆)alkyl group, or 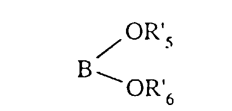 forms a pinanediol boronic ester,
is reacted with a compound of formula (III): 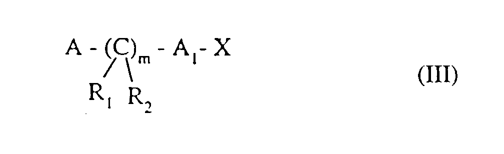 wherein A, A₁, R₁, R₂ and m are as defined for formula (I),
X represents a chlorine atom or a hydroxy group,
to yield a compound of formula (IV): 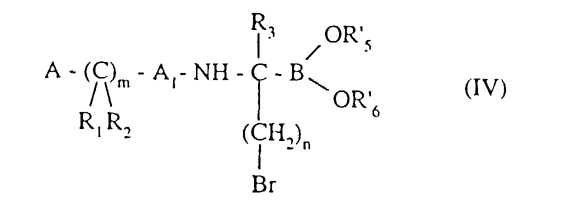 wherein A, A₁, R₁, R₂, R₃, R'₅, R'₆, m and n are as defined hereinbefore,
which compound of formula (IV) may be converted, depending upon the nature of the R₄ group that it is desired to obtain:
- **either** into the corresponding cyano compound by the action of copper cyanide, and then by reaction in an alcoholic medium in the presence of an acid followed by the action of ammonia into the corresponding amidino compound of (I/a), a particular case of the compounds of formula (I): 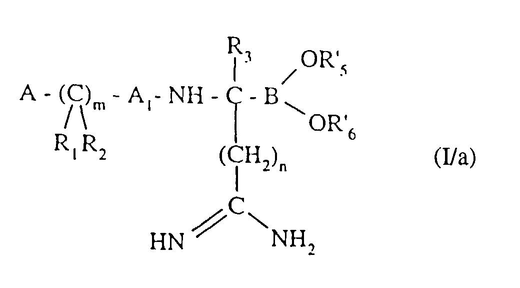 wherein A, A₁, R₁, R₂, R₃, R'₅, R'₆, m and n are as defined hereinbefore,
the amidino function of which is substituted if desired,
- **or** into the corresponding azido compound by the action of sodium azide, and then by catalytic hydrogenation into the corresponding amino compound of formula (I/b), a particular case of the compounds of formula (I): 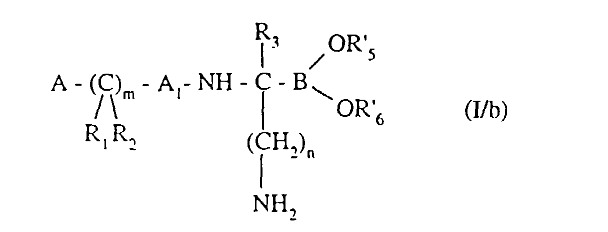 wherein A, A₁, R₁, R₂, R₃, R'₅, R'₆, m and n are as defined hereinbefore,
the amine function of which compound of formula (I/b) is substituted if desired,
and the amino group of which is converted, if desired,
- into a *guanidino group* by reaction with cyanamide,
to yield the compound of formula (I/c), a particular case of the compounds of formula (I): 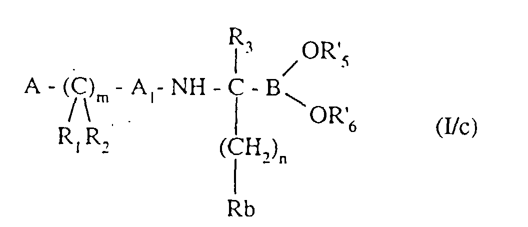 wherein A, A₁, R₁, R₂, R₃, R'₅, R'₆, m and n are as defined hereinbefore and Rb represents a guanidino group, the guanidino function of which is substituted if desired,
- or into an *iminomethylamino group* by reaction with ethyl formimidate to yield the compound of formula (I/d): 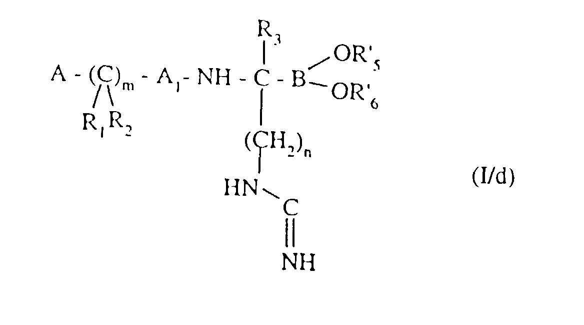 wherein A, A₁, R₁, R₂, R₃, R'₅, R'₆, m and n are as defined hereinbefore,
the amidino function of which is substituted if desired,
- **or** by reaction with an optionally substituted thiourea,
to yield the compound of formula (I/e), a particular case of the compounds of formula (I): 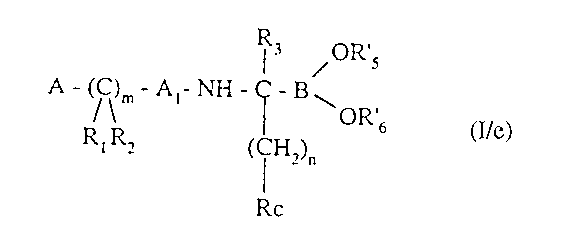 wherein A, A₁, R₁, R₂, R₃, R'₅, R'₆, m and n are as defined hereinbefore, and Rc represents an optionally substituted isothioureido group,
- **or** by reaction with a suitably protected heterocycle, with an amine substituted by a suitably protected heterocycle or with a thiol substituted by a suitably protected heterocycle,
to yield, after deprotection, the compound of formula (I/f), a particular case of the compounds of formula (I): 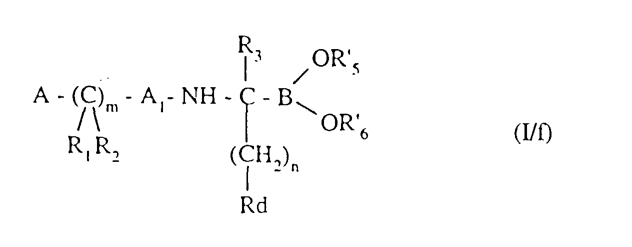 wherein A, A₁, R₁, R₂, R₃, R'₅, R'₆, m and n are as defined hereinbefore,
and Rd represents a heterocycle, an amino group substituted by a heterocycle or a mercapto group substituted by a heterocycle,
which compounds of formulae (I/a), (I/b), (I/c), (I/d), (I/e) and (I/f) are converted, if desired, using boron trichloride or phenylboronic acid, into the corresponding boronic acid of formula (I/g): 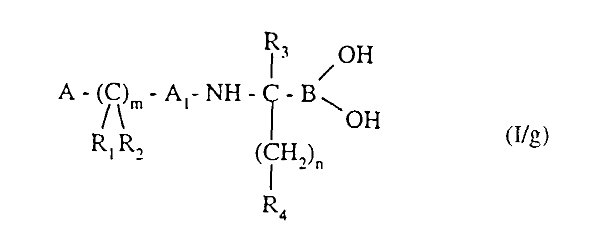 wherein A, A₁, R₁, R₂, R₃, R₄, m and n are as defined for formula (I),
which compounds of formulae (I/a) to (I/g):
- may be purified, if necessary, according to a conventional purification technique,
- are separated, where appropriate, into their isomers according to a conventional separation technique,
- are converted, if desired, into addition salts thereof with a pharmaceutically acceptable acid or base.

26. Pharmaceutical compositions comprising as active ingredient at least one compound according to any one of claims 1 to 24, on its own or in combination with one or more inert, non-toxic, pharmaceutically acceptable carriers.

27. Pharmaceutical compositions according to claim 26 for use as inhibitors of trypsine-like serine proteases.

28. Pharmaceutical compositions according to claim 27 for use as thrombin inhibitors.
